Europäisches Patentamt

(19) European Patent Office

Office européen des brevets

(11) EP 0 690 920 B1

(12) **EUROPEAN PATENT SPECIFICATION**

(45) Date of publication and mention
of the grant of the patent:
**07.01.1998 Bulletin 1998/02**

(51) Int Cl.[6]: **C12N 15/86**, C12N 15/12,
A01N 63/02, C12N 5/14,
C12N 1/21, A01H 1/06

(21) Application number: **94911998.6**

(22) Date of filing: **25.03.1994**

(86) International application number:
**PCT/GB94/00622**

(87) International publication number:
**WO 94/23047 (13.10.1994 Gazette 1994/23)**

(54) **BIOLOGICAL CONTROL AGENTS CONTAINING MOLLUSC TOXINS**

MOLLUSK-TOXINE ENTHALTENDE BIOLOGISCHE KONTROLSUBSTANZEN

AGENTS BIOLOGIQUES ANTIPARASITES CONTENANT DES TOXINES DE MOLLUSQUES

(84) Designated Contracting States:
**AT BE CH DE DK ES FR GB GR IE IT LI LU MC NL
PT SE**

(30) Priority: **26.03.1993 GB 9306295**

(43) Date of publication of application:
**10.01.1996 Bulletin 1996/02**

(73) Proprietor: **ZENECA LIMITED
London W1Y 6LN (GB)**

(72) Inventors:
• **GUEST, Philippa, Jane
Bracknell Berkshire RG12 7DH (GB)**
• **WINDASS, John, David
Wokingham Berkshire RG11 4XF (GB)**
• **SUNER, Marie-Marthe
Wokingham Berkshire RG11 4UD (GB)**
• **EARLEY, Fergus, Gerard, Paul
Southampton SO2 1RS (GB)**

(74) Representative: **Mallalieu, Catherine Louise
Intellectual Property Department
ZENECA Agrochemicals
Jealotts Hill Research Station
P.O. Box 3538
Bracknell Berkshire RG12 6YA (GB)**

(56) References cited:
**WO-A-92/15195**

• **EMBO JOURNAL vol. 9, no. 4 , April 1990 ,
EYNSHAM, OXFORD GB pages 1015 - 1020
SCOTT R. WOODWARD ET AL. 'Constant and
hypervariable regions in conotoxin propeptides'
cited in the application**
• **NATURE vol. 352, no. 6330 , 11 July 1991 ,
LONDON GB pages 85 - 88 LORNA M.D.
STEWART ET AL. 'Construction of an improved
baculovirus insecticide containing an
insect-specific toxin gene'**

SEQUENCE LISTING

(1) GENERAL INFORMATION:

(i) APPLICANT: ZENECA, LIMITED

(ii) TITLE OF INVENTION: BIOLOGICAL CONTROL AGENTS

(iii) NUMBER OF SEQUENCES: 37

(iv) CORRESPONDENCE ADDRESS:

(A) ADDRESSEE: ICI GROUP PATENTS SERVICES DEPARTMENT
(B) STREET: PO BOX 6, SHIRE PARK, BESSAMER ROAD
(C) CITY: WELWYN GARDEN CITY
(D) STATE: HERTFORDSHIRE
(E) COUNTRY: UNITED KINGDOM
(F) ZIP: AL7 1HD

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER:
(B) FILING DATE:
(C) CLASSIFICATION:

(vii) PRIOR APPLICATION DATA:

(A) APPLICATION NUMBER: GB 9306295.8
(B) FILING DATE: 22-MAR-1993

(viii) ATTORNEY/AGENT INFORMATION:

(A) NAME: BISHOP, NIGEL D
(B) REGISTRATION NUMBER: GEN AUTHN 31434
(C) REFERENCE/DOCKET NUMBER: PPD37491

(ix) TELECOMMUNICATION INFORMATION:

(A) TELEPHONE: (+44) 0707 323400
(B) TELEFAX: (+44) 0707 337454
(C) TELEX: 94028500 ICIC G

(2) INFORMATION FOR SEQ ID NO:1:

(i) SEQUENCE CHARACTERISTICS:

(A) LENGTH: 269 base pairs
(B) TYPE: nucleic acid
(C) STRANDEDNESS: single
(D) TOPOLOGY: linear

(ii) MOLECULE TYPE: cDNA

(iv) ANTI-SENSE: NO

(xi) SEQUENCE DESCRIPTION: SEQ ID NO:1:

```
TTAGATCTAA TTCACCATGA AGCTGACATG TATGATGATC GTGGCCGTGC TGTTCCTGAC      60

CGCCTGGACC TTCGCCACTG CAGACGATCC CCGCAACGGC CTGGGCAACC TGTTCTCCAA     120

CGCCCACCAC GAAATGAAGA ACCCCGAGGC ATCCAAGCTT AACAAGCGCT GGTGTAAGCA     180

GTCCGGAGAG ATGTGTAACC TGCTGGACCA GAACTGTTGT GACGGCTACT GTATCGTGCT     240

GGTGTGCACC TAGTGACGGC CGGATCCTT                                      269
```

(2) INFORMATION FOR SEQ ID NO:2:

    (i) SEQUENCE CHARACTERISTICS:

        (A) LENGTH: 78 amino acids
        (B) TYPE: amino acid
        (C) STRANDEDNESS: single
        (D) TOPOLOGY: linear

    (ii) MOLECULE TYPE: peptide

    (xi) SEQUENCE DESCRIPTION: SEQ ID NO:2:

```
Met Lys Leu Thr Cys Met Met Ile Val Ala Val Leu Phe Leu Thr Ala
1               5                   10                  15

Trp Thr Phe Ala Thr Ala Asp Asp Pro Arg Asn Gly Leu Gly Asn Leu
            20                  25                  30

Phe Ser Asn Ala His His Glu Met Lys Asn Pro Glu Ala Ser Lys Leu
        35                  40                  45

Asn Lys Arg Trp Cys Lys Gln Ser Gly Glu Met Cys Asn Leu Leu Asp
    50                  55                  60

Gln Asn Cys Cys Asp Gly Tyr Cys Ile Val Leu Val Cys Thr
65                  70                  75
```

(2) INFORMATION FOR SEQ ID NO:3:

    (i) SEQUENCE CHARACTERISTICS:

        (A) LENGTH: 84 base pairs
        (B) TYPE: nucleic acid
        (C) STRANDEDNESS: single
        (D) TOPOLOGY: linear

    (ii) MOLECULE TYPE: cDNA

    (iv) ANTI-SENSE: NO

    (xi) SEQUENCE DESCRIPTION: SEQ ID NO:3:

TTAGATCTAA TTCACCATGA AGCTGACATG TATGATGATC GTGGCCGTGC TGTTCCTGAC      60

CGCCTGGACC TTCGCCACTG CAGA      84

(2) INFORMATION FOR SEQ ID NO:4:

    (i) SEQUENCE CHARACTERISTICS:

        (A) LENGTH: 89 base pairs
        (B) TYPE: nucleic acid
        (C) STRANDEDNESS: single
        (D) TOPOLOGY: linear

    (ii) MOLECULE TYPE: cDNA

    (iv) ANTI-SENSE: NO

    (xi) SEQUENCE DESCRIPTION: SEQ ID NO:4:

GGATGCCTCG GGGTTCTTCA TTTCGTGGTG GGCGTTGGAG AACAGGTTGC CCAGGCCGTT      60

GCGGGGATCG TCTGCAGTGG CGAAGGTCC      89

(2) INFORMATION FOR SEQ ID NO:5:

    (i) SEQUENCE CHARACTERISTICS:

        (A) LENGTH: 89 base pairs
        (B) TYPE: nucleic acid
        (C) STRANDEDNESS: single
        (D) TOPOLOGY: linear

    (ii) MOLECULE TYPE: cDNA

    (iv) ANTI-SENSE: NO

    (xi) SEQUENCE DESCRIPTION: SEQ ID NO:5:

CACGAAATGA AGAACCCCGA GGCATCCAAG CTTAACAAGC GCTGGTGTAA GCAGTCCGGA      60

GAGATGTGTA ACCTGCTGGA CCAGAACTG      89

(2) INFORMATION FOR SEQ ID NO:6:

    (i) SEQUENCE CHARACTERISTICS:

        (A) LENGTH: 73 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:6:

AAGGATCCGG CCGTCACTAG GTGCACACCA GCACGATACA GTAGCTACAG TAGCCGTCTG        60

GTCACTAGGT TAC        73

(2) INFORMATION FOR SEQ ID NO:7:

    (i) SEQUENCE CHARACTERISTICS:

        (A) LENGTH: 30 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:7:

TTAGATCTAA TTCACCATGA AGCTGACATG        30

(2) INFORMATION FOR SEQ ID NO:8:

    (i) SEQUENCE CHARACTERISTICS:

        (A) LENGTH: 26 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:8:

AAGGATCCGG CCGTCACTAG GTGCAC        26

(2) INFORMATION FOR SEQ ID NO:9:

    (i) SEQUENCE CHARACTERISTICS:

        (A) LENGTH: 45 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:9:

GATCAGATCT GCAGCGGCCG CTCCAGAATT CTAGAAGGTA CCCGG        45

(2) INFORMATION FOR SEQ ID NO:10:

(i) SEQUENCE CHARACTERISTICS:

    (A) LENGTH: 45 base pairs
    (B) TYPE: nucleic acid
    (C) STRANDEDNESS: single
    (D) TOPOLOGY: linear

(ii) MOLECULE TYPE: cDNA

(xi) SEQUENCE DESCRIPTION: SEQ ID NO:10:

```
GATCCCGGGT ACCTTCTAGA ATTCCGGAGC GGCCGCTGCA GATCT                    45
```

(2) INFORMATION FOR SEQ ID NO:11:

    (i) SEQUENCE CHARACTERISTICS:

        (A) LENGTH: 19 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:11:

```
TATAAATATG CCGGATTAT                                                  19
```

(2) INFORMATION FOR SEQ ID NO:12:

    (i) SEQUENCE CHARACTERISTICS:

        (A) LENGTH: 49 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:12:

```
TATAAATATT CCGGATTATT CATACCGTCC CACCATCGGG CGCGGATCC                49
```

(2) INFORMATION FOR SEQ ID NO:13:

    (i) SEQUENCE CHARACTERISTICS:

        (A) LENGTH: 15 base pairs
        (B) TYPE: nucleic acid
        (C) STRANDEDNESS: single
        (D) TOPOLOGY: linear

(ii) MOLECULE TYPE: cDNA

(xi) SEQUENCE DESCRIPTION: SEQ ID NO:13:


CTGTTTTCGT AACAG                                                15


(2) INFORMATION FOR SEQ ID NO:14:

    (i) SEQUENCE CHARACTERISTICS:

        (A) LENGTH: 15 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:14:


CGGATTTCCT TGAAG                                                15


(2) INFORMATION FOR SEQ ID NO:15:

    (i) SEQUENCE CHARACTERISTICS:

        (A) LENGTH: 282 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:15:


GGCGCGGATC AGATCTAATT CACCATGAAG CTGACATGTA TGATGATCGT GGCCGTGCTG       60

TTCCTGACCG CCTGGACCTT CGCCACTGCA GACGATCCCC GCAACGGCCT GGGCAACCTG      120

TTCTCCAACG CCCACCACGA AATGAAGAAC CCCGAGGCAT CCAAGCTTAA CAAGCGCTGG      180

TGTAAGCAGT CCGGAGAGAT GTGTAACCTG CTGGACCAGA ACTGTTGTGA CGGCTACTGT      240

ATCGTGCTGG TGTGCACCTA GTGACGGCCG CTCCAGAATT CT                        282


(2) INFORMATION FOR SEQ ID NO:16:

    (i) SEQUENCE CHARACTERISTICS:

        (A) LENGTH: 78 amino acids
        (B) TYPE: amino acid
        (C) STRANDEDNESS: single
        (D) TOPOLOGY: linear

(ii) MOLECULE TYPE: peptide

(xi) SEQUENCE DESCRIPTION: SEQ ID NO:16:

```
Met Lys Leu Thr Cys Met Met Ile Val Ala Val Leu Phe Leu Thr Ala
1               5                   10                  15

Trp Thr Phe Ala Thr Ala Asp Asp Pro Arg Asn Gly Leu Gly Asn Leu
            20                  25                  30

Phe Ser Asn Ala His His Glu Met Lys Asn Pro Glu Ala Ser Lys Leu
        35                  40                  45

Asn Lys Arg Trp Cys Lys Gln Ser Gly Glu Met Cys Asn Leu Leu Asp
        50                  55                  60

Gln Asn Cys Cys Asp Gly Tyr Cys Ile Val Leu Val Cys Thr
65                  70                  75
```

(2) INFORMATION FOR SEQ ID NO:17:

   (i) SEQUENCE CHARACTERISTICS:

      (A) LENGTH: 77 amino acids
      (B) TYPE: amino acid
      (C) STRANDEDNESS: single
      (D) TOPOLOGY: linear

(ii) MOLECULE TYPE: peptide

(xi) SEQUENCE DESCRIPTION: SEQ ID NO:17:

```
Met Lys Leu Thr Cys Met Met Ile Val Ala Val Leu Phe Leu Thr Ala
1               5                   10                  15

Trp Thr Phe Ala Thr Ala Asp Asp Ser Ser Asn Gly Leu Glu Asn Leu
            20                  25                  30

Phe Ser Lys Ala His His Glu Met Lys Asn Pro Glu Ala Ser Lys Leu
        35                  40                  45

Asn Lys Arg Cys Ile Glu Gln Phe Asp Pro Cys Glu Met Ile Arg His
        50                  55                  60

Thr Cys Cys Val Gly Val Cys Phe Leu Met Ala Cys Ile
65                  70                  75
```

(2) INFORMATION FOR SEQ ID NO:18:

   (i) SEQUENCE CHARACTERISTICS:

      (A) LENGTH: 77 amino acids
      (B) TYPE: amino acid
      (C) STRANDEDNESS: single
      (D) TOPOLOGY: linear

(ii) MOLECULE TYPE: peptide

(xi) SEQUENCE DESCRIPTION: SEQ ID NO:18:

```
Met Lys Leu Thr Cys Met Met Ile Val Ala Val Leu Phe Leu Thr Ala
1               5                   10                  15

Trp Thr Phe Val Thr Ala Asp Asp Ser Gly Asn Gly Leu Glu Asn Leu
            20                  25                  30

Phe Ser Lys Ala His His Glu Met Lys Asn Pro Glu Ala Ser Asn Leu
        35                  40                  45

Asn Lys Arg Cys Ala Pro Phe Leu His Pro Cys Thr Phe Phe Phe Pro
        50                  55                  60

Asn Cys Cys Asn Ser Tyr Cys Val Gln Phe Ile Cys Leu
65                  70                  75
```

(2) INFORMATION FOR SEQ ID NO:19:

(i) SEQUENCE CHARACTERISTICS:

(A) LENGTH: 27 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:19:

```
Trp Cys Lys Gln Ser Gly Glu Met Cys Asn Leu Leu Asp Gln Asn Cys
1               5                   10                  15

Cys Asp Gly Tyr Cys Ile Val Leu Val Cys Thr
            20                  25
```

(2) INFORMATION FOR SEQ ID NO:20:

(i) SEQUENCE CHARACTERISTICS:

(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:20:

```
Cys Ile Glu Gln Phe Asp Pro Cys Glu Met Ile Arg His Thr Cys Cys
1               5                   10                      15

Val Gly Val Cys Phe Leu Met Ala Cys Ile
                20                  25
```

(2) INFORMATION FOR SEQ ID NO:21:

(i) SEQUENCE CHARACTERISTICS:

(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:21:

```
Cys Ala Pro Phe Leu His Pro Cys Thr Phe Phe Phe Pro Asn Cys Cys
1               5                   10                      15

Asn Ser Tyr Cys Val Gln Phe Ile Cys Leu
                20                  25
```

(2) INFORMATION FOR SEQ ID NO:22:

(i) SEQUENCE CHARACTERISTICS:

(A) LENGTH: 25 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:22:

```
Cys Lys Gly Lys Gly Ala Lys Cys Ser Arg Leu Met Tyr Asp Cys Cys
1               5                   10                      15

Thr Gly Ser Cys Arg Ser Gly Lys Cys
                20                  25
```

(2) INFORMATION FOR SEQ ID NO:23:

(i) SEQUENCE CHARACTERISTICS:

(A) LENGTH: 196 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:23:

```
TGGAATTCTG CAGACGACAG CTCCAACGGC CTGGAGAACC TGTTCTCCAA GGCCCACCAC      60

GAGATGAAGA ACCCCGAGGC CTCCAAGCTT AACAAGCGTT GCATCGAGCA GTTCGACCCC     120

TGCGAGATGA TCCGTCACAC CTGCTGCGTC GGCGTCTGCT TCCTGATGGC CTGCATCTAG     180

TGACGGCCGG ATCCTT                                                     196
```

(2) INFORMATION FOR SEQ ID NO:24:

(i) SEQUENCE CHARACTERISTICS:

(A) LENGTH: 56 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:24:

```
Ala Asp Asp Ser Ser Asn Gly Leu Glu Asn Leu Phe Ser Lys Ala His
1               5                   10              15

His Glu Met Lys Asn Pro Glu Ala Ser Lys Leu Asn Lys Arg Cys Ile
            20                  25              30

Glu Gln Phe Asp Pro Cys Glu Met Ile Arg His Thr Cys Cys Val Gly
            35                  40              45

Val Cys Phe Leu Met Ala Cys Ile
    50                  55
```

(2) INFORMATION FOR SEQ ID NO:25:

(i) SEQUENCE CHARACTERISTICS:

(A) LENGTH: 57 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:25:

```
Ala Asp Asp Pro Arg Asn Gly Leu Gly Asn Leu Phe Ser Asn Ala His
1               5                   10                  15

His Glu Met Lys Asn Pro Glu Ala Ser Lys Leu Asn Lys Arg Trp Cys
            20                  25                  30

Lys Gln Ser Gly Glu Met Cys Asn Leu Leu Asp Gln Asn Cys Cys Asp
        35                  40                  45

Gly Tyr Cys Ile Val Leu Tyr Cys Thr
        50                  55
```

(2) INFORMATION FOR SEQ ID NO:26:

    (i) SEQUENCE CHARACTERISTICS:

        (A) LENGTH: 111 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:26:

```
TGGAATTCTG CAGACGACAG CTCCAACGGC CTGGAGAACC TGTTCTCCAA GGCCCACCAC     60

GAGATGAAGA ACCCCGAGGC CTCCAAGCTT AACAAGCGTT GCATCGAGCA G            111
```

(2) INFORMATION FOR SEQ ID NO:27:

    (i) SEQUENCE CHARACTERISTICS:

        (A) LENGTH: 106 base pairs
        (B) TYPE: nucleic acid
        (C) STRANDEDNESS: single
        (D) TOPOLOGY: linear

    (ii) MOLECULE TYPE: cDNA

    (xi) SEQUENCE DESCRIPTION: SEQ ID NO:27:

```
AAGGATCCGG CCGTCACTAG ATGCAGGCCA TCAGGAAGCA GACGCCGACG CAGCAGGTGC     60

GACGGATCAT CTCGCAGGGG TCGAAGTGCT CGATGCAACG CTTGTT                  106
```

(2) INFORMATION FOR SEQ ID NO:28:

    (i) SEQUENCE CHARACTERISTICS:

        (A) LENGTH: 27 base pairs
        (B) TYPE: nucleic acid
        (C) STRANDEDNESS: single
        (D) TOPOLOGY: linear

(ii) MOLECULE TYPE: cDNA

(xi) SEQUENCE DESCRIPTION: SEQ ID NO:28:

TGGAATTCTG CAGACGACAG CTCCAAC 27

(2) INFORMATION FOR SEQ ID NO:29:

   (i) SEQUENCE CHARACTERISTICS:

      (A) LENGTH: 26 base pairs
      (B) TYPE: nucleic acid
      (C) STRANDEDNESS: single
      (D) TOPOLOGY: linear

   (ii) MOLECULE TYPE: cDNA

   (xi) SEQUENCE DESCRIPTION: SEQ ID NO:29:

AAGGATCCGG CCGTCACTAG ATGCAG 26

(2) INFORMATION FOR SEQ ID NO:30:

   (i) SEQUENCE CHARACTERISTICS:

      (A) LENGTH: 45 base pairs
      (B) TYPE: nucleic acid
      (C) STRANDEDNESS: single
      (D) TOPOLOGY: linear

   (ii) MOLECULE TYPE: cDNA

   (xi) SEQUENCE DESCRIPTION: SEQ ID NO:30:

AATTGCGGCC GGATCCAAGC TTGAGCTCGA ATTCCTGCAG ATCTG 45

(2) INFORMATION FOR SEQ ID NO:31:

   (i) SEQUENCE CHARACTERISTICS:

      (A) LENGTH: 27 amino acids
      (B) TYPE: amino acid
      (C) STRANDEDNESS: single
      (D) TOPOLOGY: linear

   (ii) MOLECULE TYPE: peptide

   (xi) SEQUENCE DESCRIPTION: SEQ ID NO:31:

```
Cys Lys Ser Pro Gly Ser Ser Cys Ser Pro Thr Ser Tyr Asn Cys Cys
1               5               10                  15

Arg Ser Cys Asn Pro Tyr Thr Lys Arg Cys Tyr
        20              25
```

(2) INFORMATION FOR SEQ ID NO:32:

(i) SEQUENCE CHARACTERISTICS:

(A) LENGTH: 25 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:32:

```
Cys Lys Gly Lys Gly Ala Ser Cys His Arg Thr Ser Tyr Asp Cys Cys
1               5               10                  15

Thr Gly Ser Cys Asn Arg Gly Lys Cys
        20              25
```

(2) INFORMATION FOR SEQ ID NO:33:

(i) SEQUENCE CHARACTERISTICS:

(A) LENGTH: 38 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:33:

```
Ala Asp Cys Val Gly Asp Gly Gln Arg Cys Ala Asp Trp Ala Gly Pro
1               5               10                  15

Tyr Cys Cys Ser Gly Tyr Tyr Cys Ser Cys Arg Ser Met Pro Tyr Cys
        20              25                  30

Arg Cys Arg Ser Asp Ser
        35
```

(2) INFORMATION FOR SEQ ID NO:34:

(i) SEQUENCE CHARACTERISTICS:

(A) LENGTH: 38 amino acids
(B) TYPE: amino acid
(C) STRANDEDNESS: single

(D) TOPOLOGY: linear

(ii) MOLECULE TYPE: peptide

(xi) SEQUENCE DESCRIPTION: SEQ ID NO:34:

```
Ala Asp Cys Val Gly Asp Gly Gln Lys Cys Ala Asp Trp Phe Gly Pro
1               5                   10                  15
Tyr Cys Cys Ser Gly Tyr Tyr Cys Ser Cys Arg Ser Met Pro Tyr Cys
            20                  25              30
Arg Cys Arg Ser Asp Ser
            35
```

(2) INFORMATION FOR SEQ ID NO:35:

(i) SEQUENCE CHARACTERISTICS:

(A) LENGTH: 28 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:35:

```
Val Gly Cys Glu Glu Asp Pro Met Asn Cys Lys Gly Lys Gln Ala Lys
1               5                   10                  15
Pro Thr Cys Cys Asn Gly Val Cys Asn Cys Asn Val
            20                  25
```

(2) INFORMATION FOR SEQ ID NO:36:

(i) SEQUENCE CHARACTERISTICS:

(A) LENGTH: 30 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:36:

```
Ala Cys Ala Glu Thr Gly Ala Val Cys Val His Asn Asp Glu Cys Cys
1               5                   10                  15
Ser Gly Ala Cys Ser Pro Ile Phe Asn Tyr Cys Leu Pro Gln
            20                  25              30
```

(2) INFORMATION FOR SEQ ID NO:37:

(i) SEQUENCE CHARACTERISTICS:

(A) LENGTH: 37 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:37:

```
Gln Cys Ile Gly Asn Gly Gly Arg Cys Asn Glu Asn Val Gly Pro Pro
1               5                   10                  15

Tyr Cys Cys Ser Gly Phe Cys Leu Arg Gln Pro Gly Gln Gly Tyr Gly
            20                  25                  30

Tyr Cys Lys Asn Arg
            35
```

**Description**

This invention relates to DNA sequences encoding proteins which are toxic to insect and like pests of agriculture and to biological control agents having a genome comprising a heterologous gene under the control of a promoter for the expression of at least one of the said peptides. In particular this invention relates to biological control agents and more particularly to biological control agents comprising a heterologous gene capable of expressing a peptide or small protein (hereinafter "protein") which is toxic to insects, the said protein being or being functionally equivalent to, a protein derived from a mollusc.

By the term biological control agent is meant any viral, prokaryotic or eukaryotic organism which when brought into association with an insect is capable of infecting the insect and interfering with the normal biochemical processes and leading ultimately to the death of the insect. Suitable biological control agents within the scope of the invention include those based on bacterial, viral and fungal pathogens of insects. Bacterial pathogens include for example Bacillus species such as B.thuringiensis, B.cereus and the like. Suitable insect viral pathogens include baculoviruses and entomopoxviruses, such as Autographa californica and Amsacta moorei and the like. Fungal pathogens of insects include for example Beauvaria species such as B. bassiana.

Viruses are particularly preferred, especially those wherein the expression of the heterologous gene is under the control of a highly efficient promoter. In the case of baculoviruses, such as Autographa californica, the promoter is that for polyhedrin gene. In the case of entomopoxvirus virus, such as Amsacta moorei, the promoter is that for the spheroidin gene.

Alternatively the biological control agent can be a genetically modified plant endophyte in which the genome has been altered to incorporate a gene which is capable of expressing protein which is toxic to insects, the said protein being or being functionally equivalent to, a protein derived from a mollusc. When such an endophyte is brought into association with a plant the protein may be expressed by the endophyte within the plant and exert toxic effects on insects feeding on or dwelling within the plant.

In a further variation the biological control agent can be a plant itself, particularly a crop plant being grown for food or fibre products, in which the plant genome has been modified by incorporation of a gene which is capable of expressing a protein which is toxic to insects, the said protein being, or being functionally equivalent to, a protein derived from a mollusc.

In recent years the toxins of members of the Conus genus of marine gastropods ("Cone Snails") have been recognised as a rich source of novel low molecular weight neuroactive peptides with a very diverse range of biological effects (Olivera et al. (1990) Science 249 257-263, Olivera (1991) J.Biol.Chem. 266 22067-22070). Members of this genus have clearly become adapted to hunt both vertebrate (fish) and invertebrate (molluscs, worms and crustacea) prey by developing highly specialised and often highly selective peptide toxins from a small number of basic forms or frameworks, viz:

CC...C...C,     C...C...CC...C...C,     CC...C...C...CC

where C represents a cysteine residue and "..." indicates short tracts of amino acids between 3 and 10 amino acids long (Olivera et al. (1990) Science 249 257-263). Different families of conotoxins (e.g. the $\alpha$-conotoxins, $\mu$-conotoxins and the w-conotoxins), distinguished primarily by the pattern of the cysteine residues they contain, can thus be recognised. Some of these families, typified by the $\alpha$-conotoxins, share very similar structures and all appear to act by antagonism of nicotinic acetylcholine receptors. Others, typified by the 1-conotoxins are rather more diverse in structure and, in fact, through similarity searches have been recognised to be structurally related to a much larger family of peptides based on the presence of the characteristic w-conotoxin-type cysteine motif and often the location of "bend promoting" amino acids (glycine and proline). Included in this larger family are such species as: the "King Kong" family of conotoxins (Hillyard et al. (1989) Biochemistry 28 358-361, Woodward et al. (1990) EMBO J. 9 1015-1020), spider toxins such as $\mu$-agatoxins, scorpion toxins such as Curtatoxin 2 and Buthus Pep2, antimicrobial proteins such as MjAMP1 derived from the seed of Mirabilis jalapa (Cammue et al. (1992) J.Biol.Chem. 267 2228-2233) and even a peptide of unknown function (CTL) encoded by the baculovirus AcMNPV (Eldridge et al. J.Virology (1992) 6563-6571). This relationship is shown in the following list of sequences, and in Tables 1 and 2:

| | | |
|---|---|---|
| Cono-KK-0 | W**C**KQS**G**EM**C**NLLDQN--**CC**D**G**-Y**C**IVLV-------**C**T | [Sequence ID-19] |
| Cono-KK-1 | **C**IEQFDP**C**EMIRHT--**CC**V**G**V-**C**FLMA-------**C**I | [Sequence ID-20] |
| Cono-KK-2 | **C**APFLHP**C**TFFFPN--**CC**N-SY**C**VQFI-------**C**L | [Sequence ID-21] |
| Cono-GVIA | **C**KSP**G**SS**C**SPTSYN--**CC**R-S-**C**NPYTK-----R**C**Y | [Sequence ID-31] |
| Cono-MVIIB | **C**KGK**G**AS**C**HRTSYD--**CC**T**G**S-**C**NRG-K------**C** | [Sequence ID-32] |
| $\mu$-Agatoxin 3 | AD**C**VGD**G**QR**C**AD-WAGPY**CC**S**G**YY**C**SCR--SMPY**C**R**C**RSDS | [Sequence ID-33] |
| Curtatoxin 2 | AD**C**VGD**G**QK**C**AD-WFGPY**CC**S**G**YY**C**SCR--SMPY**C**R**C**RSDS | [Sequence ID-34] |

(continued)

| Buthus Pep 2 | VG**C**EED**P**MN**C**KGKQAKPT**CC**N**G**V-**C**N----------**C**-NV | [Sequence ID-35] |
|---|---|---|
| CTL | A**C**AET**G**AV**C**VHNDE---**CC**S**G**A-**C**SPIF---NY--**C**LPQ | [Sequence ID-36] |
| Mj-AMP1 | Q**C**IGN**G**GR**C**NENVGPPY**CC**S**G**F-**C**L-RQPGQGYGY**C**KNR | [Sequence ID-37] |
| ("--" indicates a direct bond between adjacent amino acid residues) | | |

Whilst, the w-conotoxins themselves are considered to act via voltage gated Ca2+ channels, the mechanisms of action of the more distantly related family members is often different or presently unknown. As shown in Table 1 and Table 2, such is the diversity in amino acid sequence of the members of this large family of w-conotoxin related peptides that it would be quite remarkable if they all shared similar targets. Rather the diversity of biological activities of these proteins might be taken as an indication that the basic w-conotoxin molecular framework is a particularly versatile basis upon which to construct effective small protein/peptide ligands. Given the activities and origins (spiders and scorpion venoms) of some of the more distantly related members of the w-conotoxin family it is not surprising that some can display insecticidal activety. A priori however, given the marine environment which they inhabit, there is little reason to believe that insect active conotoxins would be a particular advantage to cone snails. Not unsurprisingly therefore the issue as to whether conotoxins might provide a useful natural source of insect active or insect selective neuropeptides remains an unresolved and even largely unadressed issue. Thus, for example, in only one report that we are aware of has a group of closely related toxins isolated from the venom of Conus textile neovaricus, including the "King Kong" toxin (designated TxIA), been assessed for insecticidal activity by injection studies in blowfly (Sarcophaga falculata) larvae (Fainzilber et al. (1991) Eur.J.Biochem. 202 589-595). No activity was detected, whereas potent effects on molluscs were apparent at much lower doses.

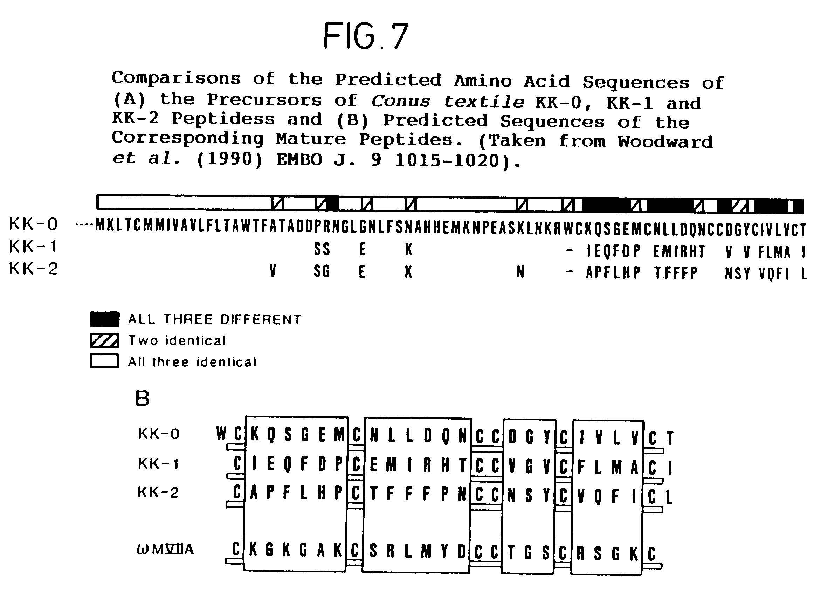

Notwithstanding the report of Fainzilber et al. we initiated an investigation intended to establish whether the mollusc and crustacean active "King Kong" conotoxin (Hillyard et al. (1989) Biochemistry), later designated KK-0 [SEQ 1D 16] (Woodward et al. (1990) EMBO J. 9 1015-1020), and two related peptides KK-1 [SEQ 1D 17] and KK-2 [SEQ 1D 18] reported at the Conferences Jacques Monod on Toxines Animales (Aussois, France) on 24-28 October 1988 had any activity in insect systems. As detailed in Example 1 below, synthetic peptides corresponding to the reported sequences of KK-0, KK-1 and KK-2 (see Figure 7) were prepared and assessed by injection into specimens of adult Periplanata americana (Order: Dictyoptera) and larval Heliothis virescens and Trichoplusia ni (Order: Lepidoptera). Both the preparations KK-0 and KK-1 showed distinct insecticidal and/or paralysis inducing activity in each of the species tested.

In light of the results of Fainzilber et al. in which TxIA (equivalent to KK-0) had been injected into Sarcophaga falculata (Order: Diptera) larvae, without effect, we considered that there might still be some doubt whether the insecticidal effects we had seen were an intrinsic property of KK-0 and/or KK-1. Although the synthetic peptide preparations had been extensively purified, some difficulties had been experienced in their chemical synthesis because of their high cysteine content, resultant tendency to aggregate and likely propensity to form incorrectly folded structures. The insecticidal activity we had seen might therefore have been a consequence of the synthetic methods used.

We have therefore extended our investigation of the insecticidal properties and potential of the Conus textile derived proteins KK-0 and KK-1 by expressing genes encoding their precursors in baculovirus (AcMNPV) expression systems. Here we report for the first time the discovery that a gene encoding the KK-0 precursor is capable of significantly shortening the time a baculovirus takes to incapacitate susceptible insect hosts. This improvement in the speed of action of the baculovirus arises from a characteristic process of paralysis followed by death and results in a significant reduction in the size of susceptible lepidopteran larvae fed with KK-0 recombinant viruses when compared to control larvae fed with wild type larvae at the same developmental stage. These effects result in a clear crop protective effect when susceptible larvae become infected by eating host plants which have been sprayed with suitable virus preparations. This crop protective effect is demonstrably better than can be achieved with similar doses of wild type virus.

Such improvements in the properties of baculoviruses were obtained using a synthetic gene [SEQ 1D 1] designed to encode an mRNA molecule which would in turn encode the presumed natural precursor of the "King Kong" (KK-0) conotoxin: a 78 amino acid protein which is likely to enter the secretion pathway during synthesis and may initially be produced as a pro-toxin which is subsequently processed to produce mature KK-0, at least in Conus textile (Woodward et al. (1990) EMBO J. 9 1015-1020). The synthetic gene we used for this work was designed without regard for the natural nucleotide sequence of the Conu stextile KK-0 gene. Rather the gene was designed to ensure that, although it encoded the natural KK-0 precursor, it was likely to be both efficiently expressed in insect cell systems and was convenient to manipulate (see details in Example 2). Though we have not therefore used a natural KK-0 gene or cDNA from Conus textile there are, however, no grounds to anticipate that such a sequence, or another synthetic gene capable of encoding the KK-0 precursor, would not be equally effective in enhancing the speed of action of a baculovirus host. The synthetic gene (sKK-0) we have employed actually has only 77% nucleotide sequence identity the coding sequence of natural KK-0 mRNA.

A superficially surprising aspect of our investigation of the properties of recombinant baculoviruses based upon Autographa californica Multiply Enveloped Nuclear Polyhedrosis Virus (AcMNPV) to express the sKK-0 gene was the discovery that the properties of the viruses are very dependent upon the promoter which is used to express the gene. Polyhedrin plus (occluded) viruses which carry the sKK-0 gene in a position such that it will be transcribed from the powerful very late p10 promoter show no enhancement in biological activity. By contrast both polyhedrin plus (polyhedrin+ or pol+) and polyhedrin minus (polyhedrin- or pol- / non-occluded) viruses in which the alternative very late polyhedrin promoter drives sKK-0 expression deliver clearcut improvements in speed of action. Literature precedent would have suggested that either the p10 (Stewart et al. (1991) Nature 352 85-88, McCutchen et al. (1991) Biotechnology 9 848-852) or the polyhedrin promoter (Tomalski & Miller (1991) Nature 352 82-85 & (1992) Biotechnology 10 545-549) would have been expected to be able to confer adequate expression levels on insecticidal gene products. Recently published patent applications also appear to presume, without providing any substantive proof, that these promoters will be able to produce enhancements in insecticidal activity when expressing either "Insecticidal Toxins from the Parasitic Wasp Bracon hebetor" (WO 93/18145) or "Insecticidal Toxins from Plectreurys tristis" (EP 0 556 160 A2). Whilst we do not know why the sKK-0 gene does not cause insecticidal effects when expressed in recombinant AcMNPVs from the p10 promoter, amongst the most likely explanations might be differential stability effects on the hybrid polyhedrin/sKK-0 and p10/sKK-0 mRNAs. There are no grounds to conclude that similar effects would or would not be seen with another gene encoding KK-0 but having a different nucleotide sequence. Similarly there are no a priori reasons to consider that such effects would be unique to the KK-0 gene system. We conclude that the baculovirus art has not reached the stage of predicatability with respect to which promoter/toxin gene combinations will deliver the hoped for improvements in virus efficacy.

In contrast with our success in enhancing the speed of action of baculoviruses (AcMNPV) by conferring upon them the capacity to express a gene encoding the precursor of KK-0, we have to date seen no similar improvements with viruses designed to express the KK-1 precursor (see Example 14). Again this result would appear somewhat surprising given both the encouraging results which had been obtained upon injection of the chemically synthesised sample of KK-1 and the fact that the identity in the sequence of the presumed signal sequence encoding region of the precursors of KK-0 and KK-1 (Woodward et al.) had enabled us to use precisely the same gene sequence to encode this portion of the KK-1 precursor as had previously been used successfully with the sKK-0 gene. Based on our experiences with the different capacities of the polyhedrin and p10 promoters to drive sKK-0 expression we had also elected to assemble a polyhedrin promoter/sKK-1 expression construct for our work with the sKK-1 gene to optimise the chances of achieving reliable expression. Whilst we consider the work we have undertaken with sKK-1 insufficient to conclude definitively that KK-1 is incapable of enhancing the efficacy of recombinant baculovirus insecticides, given the appropriate genetic constructs, we nevertheless believe that the results we have obtained underlines the fact that one cannot presume that genes encoding any conotoxin or conotoxin-related peptide will have the capacity to enhance baculovirus activity. Such a conclusion is consistent with studies with the Ctl (Conotoxin-like) gene, which encodes a natural "conotoxin-like" peptide expressed by AcMNPV but which appears to have no biological effect so far as can be established by gene ablation studies (Eldridge et al. J.Virology (1992) 6563-6571). As shown in Table 1 and Table 2, although both CTL and KK-1 are clearly members of the w-conotoxin family, both clearly only have a low absolute level of identity with KK-0 (<40%) or any other member of the family. Indeed the seed derived anti-microbial protein Mj-AMP1 shares as much similarity with other members of the family. Thus, whilst this work teaches that genes encoding conotoxins and conotoxin-like peptides are a potentially fruitful area in which to seek species capable of enhancing baculovirus activity it cannot be taken to indicate that it is obvious that any gene so categorised will be effective.

In considering the mode of action by which KK-0 acts to incapacitate insect pests, it must be considered probable that it recognises an equivalent target protein in both insects and molluscs. Given the evolutionary distance between molluscs, crustacea and insects it would however be very surprising if the structure of the KK-0 target in insects was precisely identical to that found in the natural marine prey of Conus textile. It is therefore unlikely that KK-0 will have evolved to be the perfect ligand for the insect target. There is therefore a strong probability that the KK-0 protein could be further enhanced as an insecticidal agent by enabling it to evolve greater specificity for insect target proteins as opposed to molluscan or crustacean targets. Such evolution could be achieved by a variety of protein engineering approaches which will be familiar to those skilled in the art. Potentially the most powerful such method would obviously be one of the peptide library methods which have been developed in recent years. Such approaches teach relatively straightforward methods for randomising up to six amino acids simultaneously and selecting enhanced specificity variants from the population of peptides generated (up to $6.4 \times 10^7$ variants). Six amino acids would comprise 6/27ths or 22% of the KK-0 peptide. We therefore assert that genes encoding a protein with up to:-

$$100 - 22 = 78\% \text{ homology}$$

with KK-0 would be both a sensible and practical range in which to seek an enhanced, insect or even lepidopteran selective, variant of KK-0. Such variants are therefore considered to be included within the scope of the present invention.

PCT/US92/01503 (FMC Corporation) describes insecticidally effective peptides isolatable from *Diguetia* spider venom and DNA encoding the same.

Nature, Vol. 352, No. 6330, 11 July 1991, pages 85-88, L.M.D Stewart et al. describes the modification of baculovirus insecticide by inserting a gene encoding an insect - specific toxin from the venom of the North African Scorpion, *Androctomus autralis* Hector (AaHIT).

The present invention thus provides in a first aspect the use of synthetic preparations of the Conus textile KK-0 and KK-1 proteins to deliver insecticidal effects when administered appropriately to a susceptible insect pest.

In a second aspect the invention provides the use of a natural, synthetic or semi-synthetic gene, which encodes a precursor of the Conus textile KK-0 conotoxin, to enhance the speed of action of a recombinant baculovirus by inducing an incapacitating effect on a susceptible insect host at an earlier time than would the parental wild type baculovirus host. The baculovirus may be Autographa californica Multiply Enveloped Nuclear Polyhedrosis Virus (AcMNPV) or any other viral species for which suitable genetic engineering or manipulation techniques are available or are developed and in which there is the scope, if necessary, to optimise the context in which the KK-0 gene is placed for expression.

In a further aspect the invention provides the use of a baculovirus polyhedrin promoter in preference to the p10 promoter to drive the expression of the natural, synthetic or semi-synthetic gene encoding the precursor of the Conus textile KK-0 conotoxin.

In a yet further aspect the invention provides a design for a synthetic gene encoding a precursor of the KK-0 conotoxin which can be readily constructed by a variety of well known techniques and which, when expressed from a highly efficient promoter (including the AcMNPV polyhedrin promoter but not the AcMNPV p10 promoter), delivers a clear cut enhancement in the speed of action of a recombinant baculovirus.

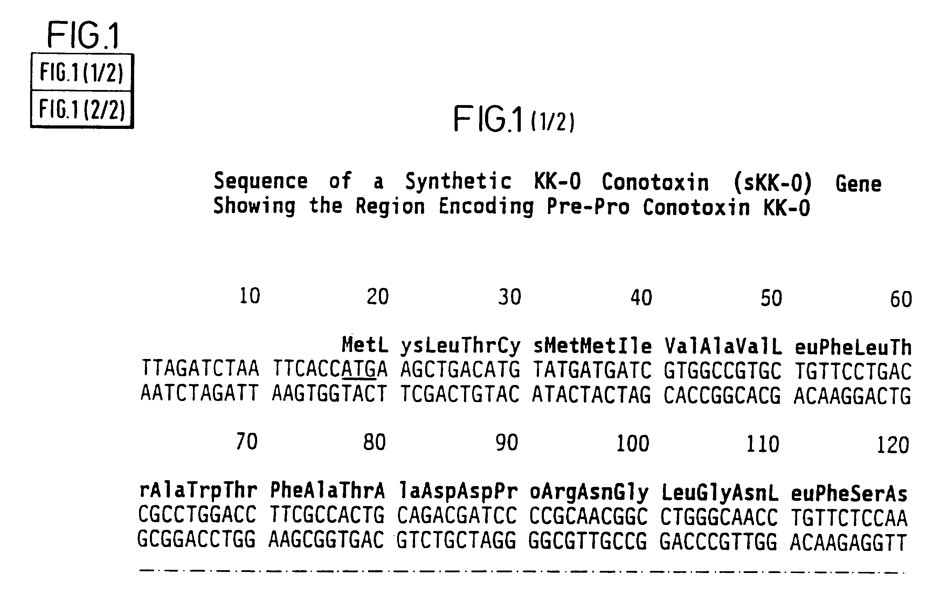

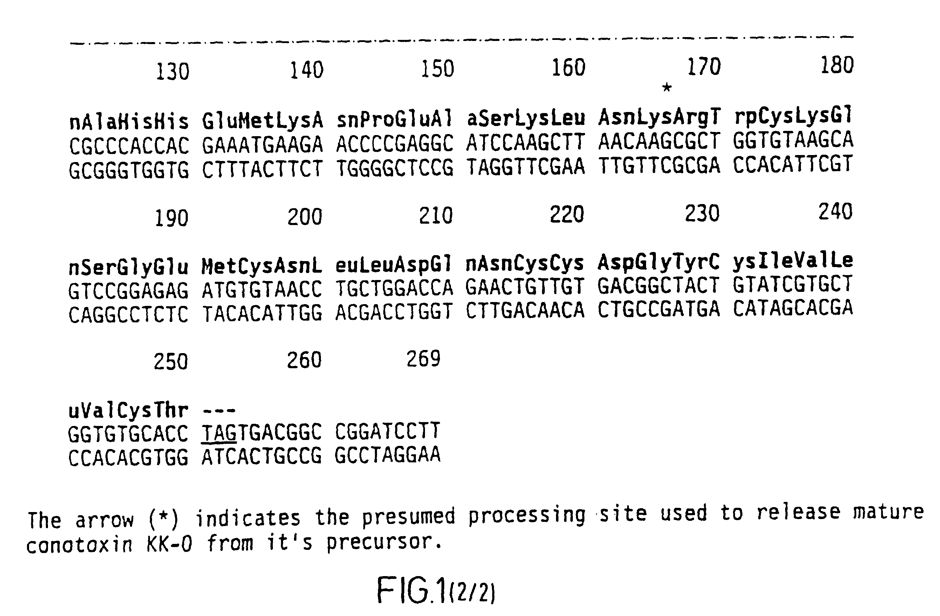

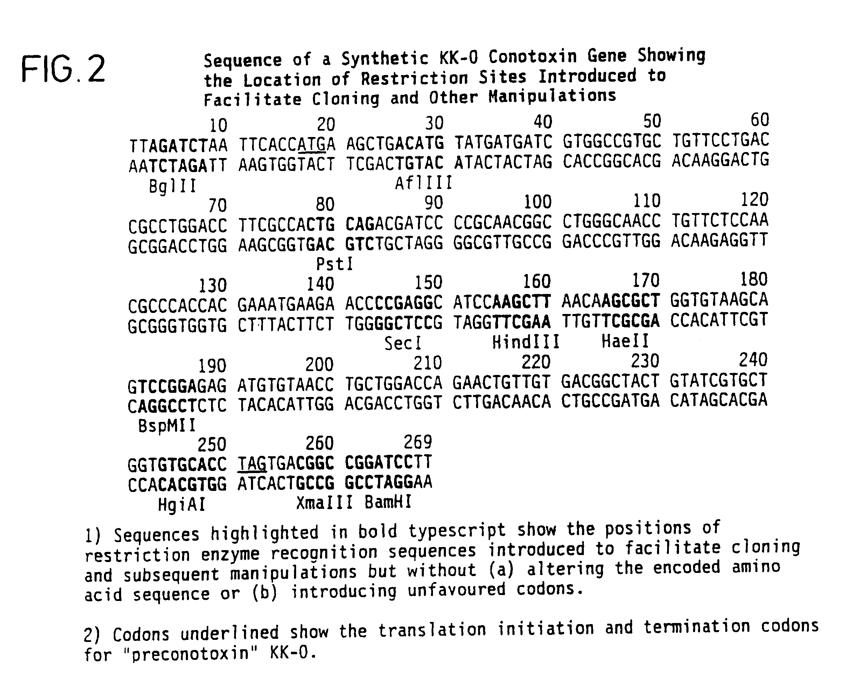

Accordingly the present invention provides a synthetic DNA encoding a gene capable of expressing a protein having the sequence shown in Figure 1, or a functional equivalent thereof, or a variant thereof permitted by the degeneracy of the genetic code. In addition the invention provides a synthetic DNA having the sequence shown in Figure 1 and Figure 2 or a functional equivalent thereof or a varient thereof permitted by the degeneracy of the genetic code.

In a further aspect the invention provides a method of controlling an insect population as set forth hereinabove wherein the toxin is a peptide expressed by the DNA sequence shown in claim 1 or claim 2 or a fragment or varient thereof prossessing a toxic effect to insects.

In addition the invention provides the use of a semi-empirical screen of expression unit structure/function (promoter/KK-0 conotoxin gene combination) to ensure that the optimal means of using the conotoxin gene to deliver enhanced efficacy of a recombinant biological control agent, whether that control agent be based on AcMNPV, any other baculovirus, an entomopoxvirus, any other entomopathogenic virus, Beauveria bassiana, Metarhizium anisopliae, any other suitable entomopathogenic fungus or any suitable plant host.

In particular the invention provides the use of any gene encoding a protein or peptide, or precursor of that protein or peptide, which shares greater than 77% similarity with that of the Conus textile KK-0 to enhance the effectiveness of an insecticidal biological control agent. Such a gene may be selected from any appropriate natural source, including but not limited to cone snails, spiders, scorpions and seeds, or may be generated by any suitable protein engineering technique.

The invention also provides a method of controlling an insect population at a locus, eg a growing plant, by administering to the insects or the locus of the insects a peptide toxic to those insects, said peptide being functionally equivalent to, and having more than 77% homology with the KK-0 or KK-1 peptides derived from a mollusc in the genus Conus, such as for example Conus textile.

In a yet further aspect the invention provides a transmission vector incorporating a DNA sequence encoding a gene capable of expressing a protein having the sequence shown in Figure 1, or a functional equivalent thereof, or a variant thereof permitted by the degeneracy of the genetic code, or alternatively incorporating a DNA having the sequence shown in Figure 1 or Figure 2 or a functional equivalent thereof or a varient thereof permitted by the degeneracy of the genetic code. A preferred transmission vector in a plasmid, for example a plasmid suitable for use in connection with the modification of the baculovirus genome, such as that known as pVL1392. Other plasmid vectors include for example that known as pAcUW21.

A plasmid based on pVL1392 incorporating the DNA sequence of Figure 1 and Figure 2 has been introduced into Escherichia coli and desposited in the National Collection of Industrial and Marine Bacteria, Aberdeen UK, under the number NCIMB 40540, on 12 March 1993.

The various aspects of the invention are illustrated by means of the following Examples.

## EXAMPLE 1

### Biological Assessment of Synthetic Preparations of "King Kong" (KK-0), KK-1 and KK-2 Peptides

Preliminary assessments of the biological activity of synthetic preparations of KK-0, KK-1 and KK-2 peptides were made in a series of injection studies using adult cockroaches (Periplaneta americana: Blattidae: Dictyoptera) and fifth-instar tobacco budworm larvae (Heliothis virescens: Noctuidae: Lepidoptera).

The proteins proved to be insoluble in distilled water and the standard aqueous buffers normally used for injection. KK-0 and KK-1 were suspended at a concentration of 10mg/ml in 0.1M ammonium bicarbonate. After gentle agitation for 1 hour the suspensions were filtered through a 0.2μm filter. A visual assessment indicated that approximately 50% of the KK-0 product was solubilised whereas substantially less of the KK-1 product dissolved. This made it difficult to accurately quantify and compare dose rates directly for the different proteins. However, it was possible to estimate the maximum doses delivered. KK-2 was extremely hydrophobic and was dissolved in DMSO at a concentration of 10 mg of crude product per ml.

Tobacco budworm larvae (TBW) were injected using a 10 μl Hamilton syringe fitted with a 15 mm 33 gauge needle. Early fifth-instar larvae with an average body mass of approximately 300 mg were injected with 1-4 μl of each treatment. Typically, there were at least 5 replicates of each treatment. Adult male cockroaches were injected using a 50 μl Hamilton syringe fitted with a 15mm 27 gauge needle. Each animal received a dose of 1-10 μl of test solution; average body mass was approximately 840 mg. Control insects were injected with an equivalent volume of the appropriate solvent.

After injection, treated insects were held individually in containers with a food supply under controlled conditions (25 C, 65% RH) for up to 72 hours. Observations were made periodically to check for any unusual symptomology.

Tobacco budworm

Injection of both KK-0 and KK-1 into H.virescens larvae resulted in a characteristic "flaccid paralysis" (Table III). Typically, symptoms were observed almost immediately after injection with larvae appearing to become "narcotised". Affected larvae were unable to stand and/or take a coordinated step. Furthermore, they were unable to reinvert when placed upon their back and were capable only of limited feeble movements of the mouthparts and claspers in response to stimulation (gentle prodding with a wooden cocktail stick). At the peak of the "narcosis", affected larvae were limp and pliable. These effects were relatively short-lived, however, and full recovery had normally occurred by 5-10 minutes after injection. All larvae were alive and well at 24 hours after treatment (24 HAT). Injection of higher doses led to an increase in both the severity and duration of the symptoms. The symptomology appeared to be more prononunced with KK-0.

No abnormal effects were observed following injection of KK-2 protein into H.virescens larvae at a single rate equivalent to a maximum dose of approximately 10 μg protein per larva (ie 33 μg protein/mg larva). Cockroaches

All three proteins produced distinctive and debilitating effects in cockroaches (Table IV). In each case, the severity of the effects increased with the volume of protein suspension injected.

Injection of KK-0 protein suspension into cockroaches initially caused severe tremoring followed by a loss of coordination, paralysis and knockdown. The effects were observed initially in the back legs but rapidly spread to the middle and finally the front legs leading to the collapse of the insect. Other symptoms included dorsal arching. Affected insects failed to recover and were dead by 22 HAT. Insects injected with the KK-1 protein showed similar symptomology although the effects appeared to be slightly less severe at the lowest dose of 1 μl of protein suspension.

Abnormal effects were also observed briefly in insects injected with 2 and 5 μl of the KK-2 peptide. Symptoms included arching of the back, flattening of the wings and loss of coordinated movement and occurred within a few minutes of injection. The effects appeared to be transitory and affected insects appeared to have fully recovered by 20 minutes post-injection. However, all animals in these treatments were dead at 72 HAT. No unusual symptoms were recorded in insects treated with the lowest rate (1 μl volume) and all insects were alive and well at 72 HAT.

These observations were confirmed in subsequent experiments and demonstrated that the conotoxin proteins have insecticidal activity against cockroaches and TBW larvae.

## EXAMPLE 2

### Synthetic King Kong (sKK-0) Conotoxin Gene Design

Since there was no a priori reason to believe that the codon usage of the natural KK-0 gene would be particularly advantageous for expression in insect cells, we decided to design a novel synthetic (sKK-0) gene. This was undertaken with the aid of such PCGene™ (Intelligenetics, 700 East Camino Real, Mountain View, California) programmes as TRANSL, RESTRI and MUTSITE.

Features of the sKK-0 gene were that:-

. It encoded precisely the amino acid sequence [SEQ 1D 2] for the KK-0 propeptide described by Woodward et al. through their cDNA cloning studies ((1990) EMBO J. **9** 1015-1020).

. The codons used to encode the KK-0 propeptide were selected because they are favoured or, at least, frequently encountered in insects (Drosophila melanogaster) and yeast (Saccharomyces cerevisiae) since it was anticipated that expression studies might also be undertaken in the latter organism (Ashburner et al. (1984) Develop. Genetics **4** 295-312; Ikemura (1985) Mol.Biol.Evol. 2 13-34; Sharp (1986) Nucleic Acids Research **14** 5125-5143).

. The nucleotide sequence immediately preceding the translation initiation codon (ATG) matches closely the preferred sequence for animal mRNAs defined by Kozak ((Kozak (1981) Nucleic Acids Research **9** 5233-5252; Kozak (1984) Nucleic Acids Research **12** 857-873; Kozak (1986) Cell **44** 283-292; Kozak (1987) J.Mol.Biol. **196** 947-950).

. No account at all was taken of the codons used by Conus textile to encode the KK-0 propeptide. (As a result the overall homology of sKK-0 and natural KK-0 genes is only 77.2%).

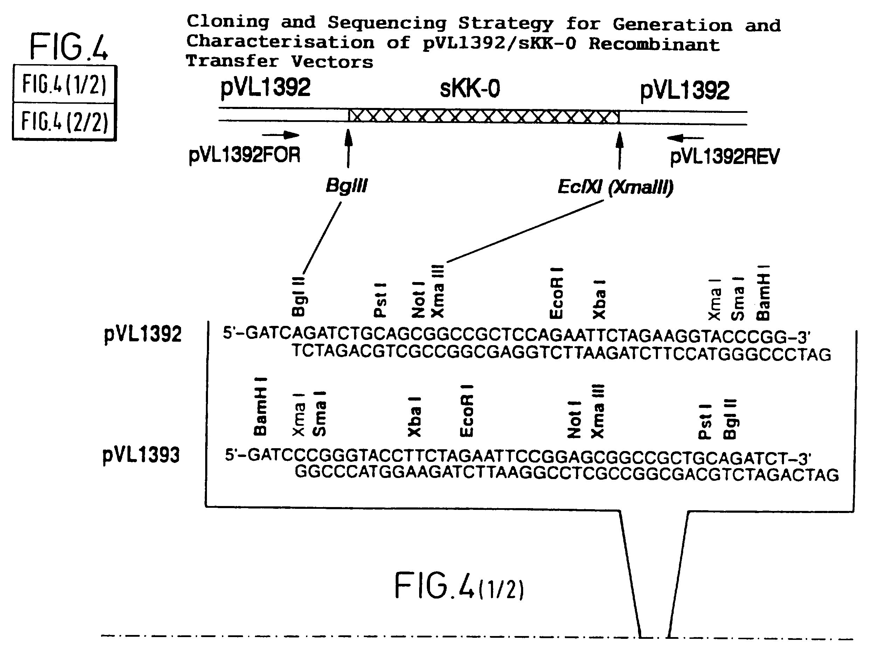

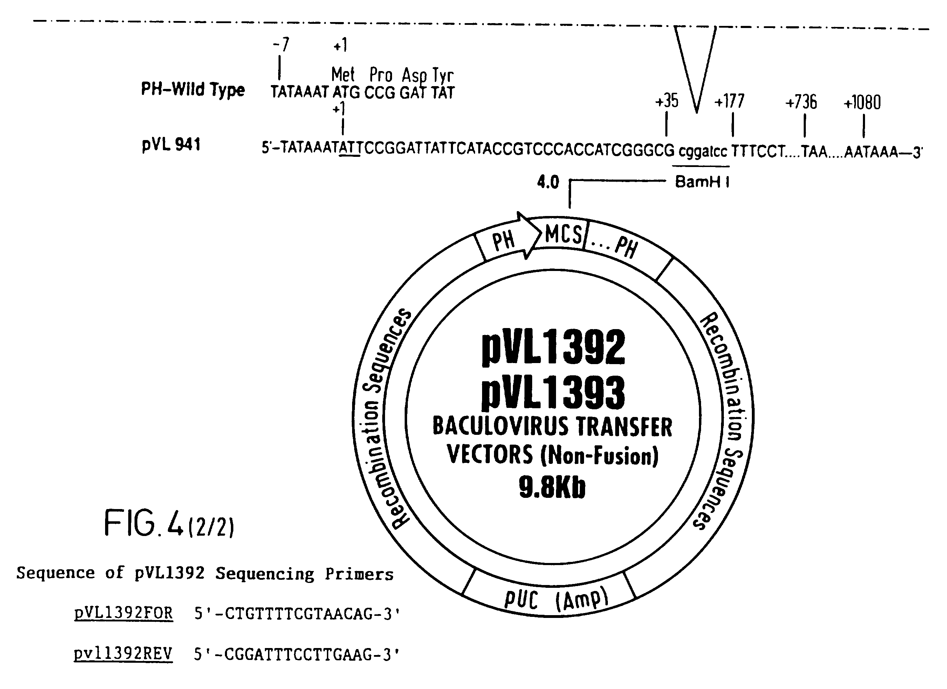

. Flanking restriction enzyme recognition sites (upstream BglII and downstream XmaIII and BamHI) were incorporated into the design to facilitate direct cloning into the initially chosen baculovirus transfer vector pVL1392 (InVitrogen Corporation, 3985 Sorrento Valley Boulevard, San Diego, California). Cloning with these enzymes was expected to result in the potential to transcribe the gene directly from the AcMNPV polyhedrin promoter carried by this transfer vector (see Figure 4).

. Several additional unique hexanucleotide recognition sequence sites were incorporated into the sKK-0 sequence at positions throughout the gene but without affecting the amino acid sequence encoded. These sites were included to facilitate recombinant characterisation and possible subsequent manipulations of the gene (see Example 14). None of the sites incorporated would result in the inclusion of particularly unfavourable codons.

The resultant sKK-0 sequence [SEQ 1D 1] is shown in Figure 1, highlighting the coding sequence for the KK-0 propeptide, and in Figure 2, highlighting the position of restriction enzyme recognition sites.

## EXAMPLE 3

### Synthesis of an sKK-0 Gene

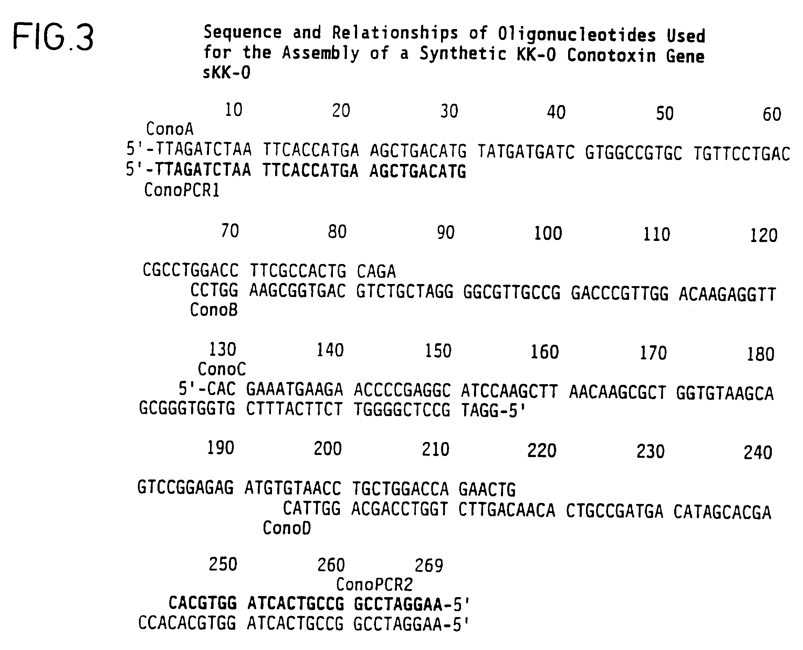

The strategy selected for assembly of the sKK-0 gene involved synthesis of overlapping complementary oligonucleotides encoding the DNA fragment shown in Figures 1 and 2 followed by DNA polymerase mediated completion of the single stranded regions and polymerase chain reaction (PCR) amplification to generate abundant quantities of the complete gene fragment (see Figure 3).

Thus 4 oligonucleotides (ConoA, ConoB, ConoC, ConoD) [SEQ 1D 3, 4, 5, 6], which together encode the complete sKK-0 DNA fragment, were synthesised by standard methods on an Applied Biosystems 380B DNA Synthesiser. Two additional shorter oligonucleotides, ConoPCR1 & ConoPCR2 [SEQ 1D 7, 8], which are identical to the 5' 30 and 28 nucleotides of ConoA and ConoD were also synthesised to use as PCR primers. Each oligonucleotide was deprotected by incubation for approx. 8 hours at 55° followed by drying down under vacuum. They were then dissolved in 200µl TE buffer (10mM Tris/HCl (pH 8.0), 1mM EDTA), concentrations measured by UV spectroscopy (1 0D$_{260}$ unit/ml. equiv-alent to approximately 20µg/ml) and diluted with TE to give approximately 10µM stocks. These were stored at -20°C until use. sKK-0 synthesis reactions then comprised:-

1) Mixing 5µl each of 10µM ConoA and ConoB in a sterile 500µl conical polypropylene microfuge tube together with 5µl 10mM dGTP, 10mM dATP, 10mM dCTP, 10mM TTP and 5µl 500mM KCl, 100mM Tris-HCl (pH 8.5), 15mM MgCl$_2$ 0.1% gelatine, making up to 49µl with water and adding 1µl (5 units) Taq DNA polymerase (Perkin/Elmer Cetus) before overlaying with 2 drops of light paraffin oil (Sigma). The tube was then capped, placed in a Techne PHC-1 Programmable Dri-Block$^R$ and subject to the following temperature cycle:-

1.1' @ 94°C

1' @ 55°C

4' @ 73°C

5 times, followed by a final incubation period of 7' @ 73°C.

2) Mixing 5μl each of 10μM ConoC and ConoD and treating exactly as in (1) above.

3) Taking 0.5μl reaction (1) products mixing with 0.5μl reaction (2) products and 5μl reaction (1) products mixed with reaction (2) products. To each mixture was then added 10μl 10μM ConoPCR1; 10μl 10μM ConoPCR2; 10μl 500mM KCl, 100mM Tris-HCl (pH 8.5), 15mM $MgCl_2$, 0.1% gelatine; 10μl 10mM dGTP, 10mM dATP, 10mM dCTP, 10mM TTP, and then making up to 99μl with water before adding 1μl (5 units) Taq DNA polymerase (Perkin/Elmer Cetus) and finally overlaying with two drops of light paraffin oil. In parallel, control reactions were set up which lacked PCR primers, reaction 1 or reaction 2 products. The reactions were then subject to the following temperature cycle in the Techne Programmable Dri-Block[R]:-

    1.1' @ 94°C
    1'   @ 68°C
    4'   @ 73°C

25 times, followed by a final incubation period of 7' @ 73°C.

Analytical agarose gel electrophoresis on 10 μl aliquots of reaction 3 products then confirmed that only in those reactions which had contained all necessary DNA fragments and oligonucleotides had an approx. 270bp fragment been produced. The remainder of each DNA fragment was then worked up by phenol extraction with an equal volume of water saturated phenol (twice) and then an equal volume of water saturated n-butanol (twice) before recovery by ethanol precipitation.

### EXAMPLE 4

### Assembly of pVL1392/sKK-0 Recombinant Transfer Vectors

After recovery, each of the above PCR products was subject to digestion with BglII and EclXI (an isoschizomer of XmaIII) restriction enzymes according to the manufacturers recommendations. In parallel an approx. 5μg aliquot of pVL1392, a commercially available baculovirus (AcMNPV) transfer vector (InVitrogen), was similarly treated. Restriction digests were then run on a preparative 0.8% agarose/Tris-acetate electrophoresis gel containing 0.5μg/ml ethidium bromide. The PCR products and the linearised vector DNA fragments were excised from the gel under UV illumination. They were then recovered by centrifugation through siliconised glass wool and ethanol precipitation. Aliquots of the isolated insert and vector DNA fragments were then mixed, together with T4 ligase in the appropriate buffer conditions (Sambrook J., Fritsch E.F. & Maniatis T. (1989) "Molecular Cloning: A Laboratory Manual". Cold Spring Harbor Press.). The ligation mixtures were then used to transform competent DH5a cells by standard methods (Sambrook et al. ibid.). Progeny transformants were selected by overnight growth on L agar plates containing 100μg/ml ampicillin.

Transformants were then screened for the presence of sequences complementary to the sKK-0 gene by colony hybridisation on HyBond-N (Amersham International) filters by standard procedures (Sambrook et al. ibid.). The hybridisation probe used was oligonucleotide ConoB, which had been 5' phosphorylated by treatment with g[32]P-ATP (Amersham International) and T4 polynucleotide kinase (Northumbria Biologicals Ltd.). Plasmid DNA was prepared from six strongly hybridising colonies. Restriction analysis of these DNAs suggested that three were pVL1392 derivatives containing inserts with approximately the size and features expected for sKK-0.

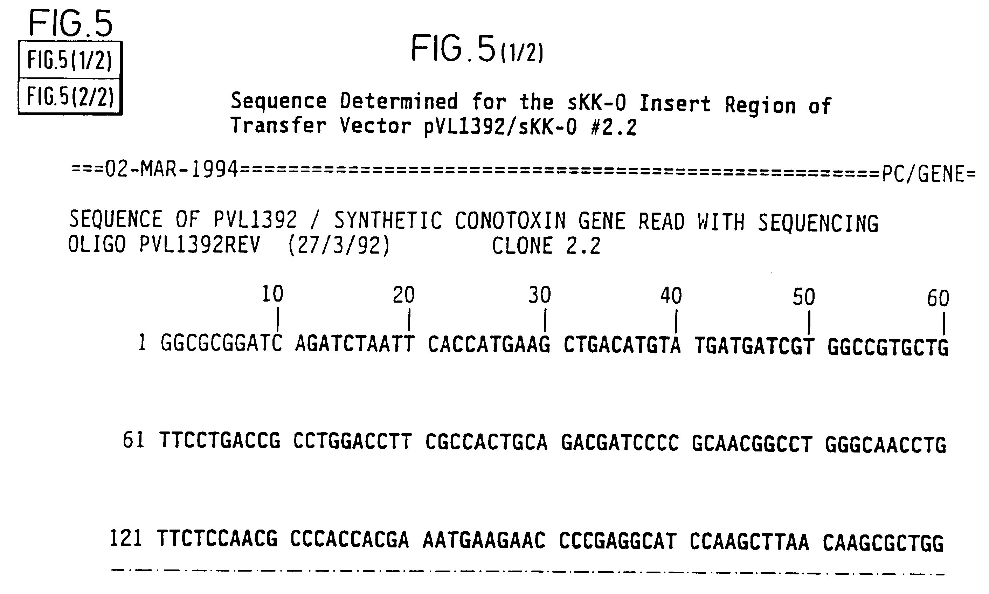

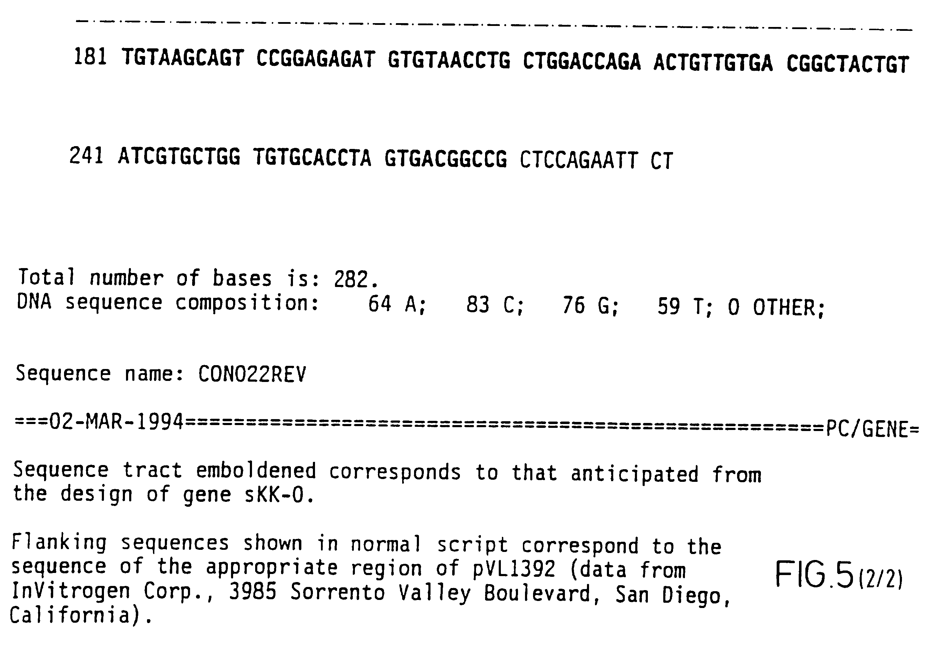

The sequence of the inserts present in the selected pVL1392 recombinants (#2.2, #5.2 & #6.1) was then checked using a Sequenase™ (USB, Cleveland, Ohio) kit and two synthetic oligonucleotide primers (pVL1392FOR and pVL1392REV) [SEQ 1D 13, 14] designed to hybridise to the regions of pVL1392 flanking the BglII and XmaIII recognition sites and to allow sequencing of any insert introduced between those sites (see Figure 4). The sequence of the insert [SEQ, 1D 15] in one of the three recombinants (#2.2) matched precisely that expected for sKK-0 cloned in the expected manner (see Figure 5). The other plasmids contained inserts with minor sequence variations (mutations).

Recombinant #2.2, a derivative of transfer vector pVL1392 containing gene sKK-0 under the transcriptional control of the AcMNPV polyhedrin promoter, was therefore selected for all subsequent studies. An E.coli DH5α culture carrying recombinant plasmid pVL1392/sKK-0 #2.2 has been deposited in the National Collection of Industrial and Marine Bacteria, Aberdeen, UK as deposit number NCIMB 40540.

### EXAMPLE 5

### Generation of Recombinant (Polyhedrin Minus) AcMNPV Carrying sKK-0

Using standard baculovirus techniques, as described for example in King & Possee (1992) "The Baculovirus Expression System: A Laboratory Guide" (Chapman & Hall), recombinant AcMNPV virus carrying sKK-0 under the transcriptional control of the polyhedrin promoter, but lacking a functional polyhedrin gene, were generated by co-transfection of Sf21 cells with approx. 200ng. Saul (Boehringer, Mannheim) linearised AcMNPV.lacZ (Possee & Howard (1987) Nucleic Acids Research **15** 10233-10248) and 1μg pVL1392/sKK-0 #2.2 DNA. Selection of recombinant virus was initially based on the inability of recombinant lacZ- virus to metabolise X-gal (GIBCO/BRL, Grand Island, New York) in plaques generated in Sf21 cell monolayer culture. Six such lacZ- virus ("CONO-A", "CONO-B", "CONO-C", "CONO-D", "CONO-D" and "CONO-E") were picked and replated on Sf21 cells to purify them to homogeneity and confirm their inability to synthesise β galactosidase. A small scale (approx. 4 ml) culture of each purified virus was then prepared by seeding $1.5 \times 10^6$ Sf21 cells in 4ml. TC100/7.5% foetal calf serum medium (both from GIBCO/BRL) in a $25cm^2$ tissue culture flask (Bibby, Stone, Staffs.)), incubation overnight at 28°C, addition of 50% of the virus recovered by picking a single purified plaque and continuing incubation at 28°C for a further 6 days. Aliquots of each of these virus stocks were then taken for bioassay (see Example 6). Parallel aliquots were used to prepare small samples of viral DNA for physical analysis (King & Possee (1992) ibid.).

Diagnostic physical analyses undertaken with the prospective sKK-0/AcMNPV recombinants were:-

a) PCR studies with sKK-0 and, in parallel, βgalactosidase gene specific primers.

b) Southern blot analysis on viral DNA treated with ClaI and BglII, fractionated by electrophoresis on a 1% agarose gel, transferred to HyBond-N filters and hybridised with an isolated, random-primed $\alpha^{32}$P-dCTP labelled, sKK-0 probe.

These studies confirmed that virus "CONO-C" contained an sKK-0 gene, with all the expected physical features, and lacked a βgalactosidase gene. The other prospective recombinant viruses lacked the sKK-0 gene.

## EXAMPLE 6

### Bioassay of Prospective AcMNPV/pVL1392/sKK-0 Recombinant Virus

The biological activity of putative AcMNPV/pVL1392/sKK-0 viruses was evaluated in a series of injection studies using late-stage Heliothis virescens larvae. Aliquots of purified non-occluded virus were injected into fourth- or fifth-instar H.virescens larvae using a 10 μl Hamilton syringe fitted with a 15 mm 33 gauge needle. Typically, at least 5 larvae were injected per treatment with a standard volume of 1 μl volume of virus suspension. Control insects were injected with an equivalent volume of AcMNPV/pVL1392/lacZ virus, AcMNPV wild type virus or sterile water. After injection, larvae were held individually on artificial diet and examined at 24-hour intervals thereafter for a further 7 days. On each assessment occasion the number of live and dead larvae were recorded. Surviving larvae were examined for any unusual symptomology and/or behavioural responses.

Initially, aliquots of the six putative AcMNPV/pVL1392/sKK-0 clones generated as described in Example 5 were tested. Only one, AcMNPV/pVL1392/sKK-0 CONO-C, exhibited any unusual effects. The biological observations confirmed the physical analyses described in Example 5 which indicated that only the CONO-C virus carried the sKK-0 gene with all the expected physical features. Further injection assays to characterise the biological properties of AcMNPV/pVL1392/sKK-0 CONO C were carried out using titred stocks of the virus.

Data comparing the biological efficacy of AcMNPV/pVL1392/sKK-0 CONO-C and AcMNPV/pVL1392/lacZ viruses against early fourth-instar H.virescens larvae are presented in Table V. At 72 hours after treatment (72 HAT), 4/11 larvae treated with the AcMNPV/pVL1392/sKK-0 CONO C showed either full or partial flaccid paralysis. Larvae classified as "fully paralysed" were moribund: unable to stand, reinvert or walk and capable only of very feeble movements of the mouthparts and claspers in response to stimulation. "Partially paralysed" larvae showed local paralysis of the mid and posterior regions of the body although the anterior region including the head and mouthparts could respond normally to stimulation. These larvae could reinvert when placed on their backs and were capable of limited locomotion although movements were slow and poorly coordinated. At 96 HAT, all larvae treated with the AcMNPV/pVL1392/sKK-0 CONO C virus were either dead or showing abnormal symptomology. In contrast, all larvae injected with the AcMNPV/pVL1392/lacZ control virus were alive and behaving normally at 96 HAT; 1/10 of these larvae had died at 120 HAT and 6/10 larvae were dead at 144 HAT. AcMNPV/pVL1392 derivatives carrying the lacZ gene have a slower speed of kill than the wild type virus. Typically, the wild type AcMNPV starts to cause signficant mortality at 120 HAT and beyond. Thus it may be concluded that the AcMNPV/pVL1392/sKK-0 CONO C construct has a faster speed of action and hence an enhanced insecticidal effect than both the AcMNPV wild type and AcMNPV/pVL1392/lacZ control viruses.

## EXAMPLE 7

### Assembly of pAcUW21/sKK-0 Recombinant Transfer Vectors

With the objective of preparing polyhedrin+ (occluded) recombinant AcMNPV/sKK-0 derivatives, which would be infectious per os and hence capable of realistic assessment for dose response effects and for evaluation for crop protective effects, we chose initially to employ the pAcUW21 transfer vector (AMS Biotechnology (UK) Ltd.). This transfer vector is a simple derivative (Possee R.D., personal communication) of the transfer vector pAcUW2b previously used successfully to prepare recombinant viruses capable of delivering an accelerated biological effect because of the expression of insect selective toxin genes under the control of the powerful late p10 promoter (Stewart et al. (1991) Nature **352** 85-88; McCutchen et al. (1991) Biotechnology **9** 848-852).

To release a suitable sKK-0 insert for introduction into pAcUW21 a 20 μg aliquot of the pVL1392/sKK-0 #2.2 plasmid DNA was subject to digestion with EcoRI and BglII restriction enzymes. In parallel, a 10 μg aliquot of pAcUW21 transfer vector DNA was similarly treated. The restriction digests were then run on a preparative 1% agarose/Tris-acetate electrophoresis gel containing 0.5μg/ml ethidium bromide. The released sKK-0 insert (approx. 282 base pairs) and the linearised vector DNA were excised from the gel under UV illumination. They were then recovered by centrifugation through siliconised glass wool and ethanol precipitation.

Aliquots of the isolated sKK-0 insert and pAcUW21 vector DNA fragments were then mixed, together with T4 ligase, in the appropriate buffer conditions (Sambrook et al. (1989) in "Molecular Cloning: A Laboratory Manual" (Cold Spring Harbor Press)). The ligation mixtures were then used to transform competent DH5α cells by standard methods (Sambrook et al. ibid.). Progeny transformants were selected by overnight growth on L agar plates containing 100 μg/ml ampicillin. Plasmid DNA was prepared from six putative recombinant colonies. Restriction analysis of these DNAs suggested that all six were pAcUW21 derivatives containing inserts with the size and features expected for sKK-0.

The sequence of the inserts present in two of the above six recombinants (#A1 & #A2) was then checked using a Sequenase™ (USB, Cleveland, Ohio) kit and two synthetic oligonucleotide primers designed to hybridise to the sKK-0 insert. The sequence of the inserts of both clones matched precisely that expected for sKK-0 cloned in the intended manner.

Recombinant #A1, a derivative of transfer vector pAcUW21 containing gene sKK-0 under the transcriptional control of the AcMNPV p10 promoter, was therefore selected for subsequent studies. This recombinant transfer vector also possesses an intact AcMNPV polyhedrin gene under the control of the natural polyhedrin promoter.

## EXAMPLE 8

### Generation of a Recombinant (Polyhedrin Plus) AcMNPV Carrying a p10 Promoter/SKK-0 Gene Expression Unit

Standard baculovirus techniques (King & Possee (1992) "The Baculovirus Expression System: A Laboratory Guide" (Chapman & Hall)) were used to generate polyhedrin+ (occluded) AcMNPV derivatives carrying the sKK-0 gene under the transcriptional control of the p10 promoter. This was accomplished by co-transfection of Sf21 cells with 200 ng.SauI (Boehringer Mannheim) linearised AcMNPV.lacZ (Possee & Howard (1987) Nucleic Acids Research 15 10233-10248) and 1 μg pAcUW21/sKK-0 recombinant #A1 DNA. Candidate recombinant AcMNPV/sKK-0 viruses were then selected by plaque purification on Sf21 cell monolayers (King & Possee (1992) ibid.) screening initially for inability of viruses to metabolise X-gal (GIBCO/BRL, Grand Island, New York) and the ability to produce polyhedra (viral occlusion bodies) as judged by microscopy. Six such lacZ- virus (AcMNPV/pAcUW21/sKK-0 #1, AcMNPV/pAcUW21/SKK-0 #2, AcMNPV/pAcUW21/sKK-0 #3, AcMNPV/pAcUW21/sKK-0 #4, AcMNPV/pAcUW21/SKK-0 #5 and AcMNPV/pAcUW21/sKK-0 #6) were picked. A second plaque assay was then performed with the above six clones on Sf21 cell monolayers to purify them to homogeneity and confirm their inability to synthesise βgalactosidase and their ability to produce polyhedra. A small scale cultures of each purified virus was then prepared as described in Example 5. Aliquots of each of these virus stocks were then taken for bioassay (see Example 9). Parallel aliquots were used to prepare small samples of viral DNA for physical analysis.

Southern blot analyses were performed on the prospective ACMNPV/pAcUW21/sKK-0 viral DNAs. These DNAs were treated with: (a) HindIII and (b) EcoRI and BamHI, fractionated by electrophoresis on a 1% agarose gel, transferred to Hybond-N filters and hybridised with an isolated, random-primed α32P-dCTP labelled, sKK-0 probe.

These studies confirmed that viruses AcMNPV/pAcUW21/SKK-0 #1, AcMNPV/pAcUW21/sKK-0 #4 and AcMNPV/pAcUW21/SKK-0 #5 each contained an sKK-0 gene, with all the anticipated physical features, lacked a βgalactosidase gene and produced polyhedrin+ virus particles.

Virus AcMNPV/pAcUW21/SKK-0 #1 was selected for all subsequent studies.

## EXAMPLE 9

### Bioassay of Prospective AcMNPV/pAcUW21/sKK-0 Recombinant Virus

The biological properties of putative AcMNPV/pAcUW21/sKK-0 viruses were evaluated in a series of injection and oral dosing studies against H.virescens. Injection assays using purified non-occluded virus stocks were carried out using the method outlined in Example 6. Oral dosing tests with purified polyhedra were undertaken using a modified version of the droplet feeding method for neonate larvae developed by Hughes and Wood ((1981) J.Invertebr. Pathol. 37, 54).

Preliminary injection studies on the six putative AcMNPV/pAcUW21/sKK-0 viruses generated as described in Example 8 indicated that only three clones (AcMNPV/pAcUW21/sKK-0 #1,#4 and #5) were carrying the sKK-0 gene. Some of the larvae treated with these viruses exhibited abnormal symptoms which initially appeared to be analagous to those observed in insects treated with the AcMNPV/pVL1392/sKK-0 CONO C virus. Subsequent studies on AcMNPV/pCUW21/sKK-0 #1, however, confirmed that these "abnormal" effects were generally poorly defined and not particularly debilitating and that AcMNPV/pAcUW21/sKK-0 #1 showed no noticeable advantage over the wild type AcMNPV in terms of speed of action or insecticidal effect. Oral dosing studies comparing the biological efficacy of AcMNPV/pAcUW21/sKK-0 #1 and the wild type AcMNPV confirmed these observations.

## EXAMPLE 10

### Preparation of AcUW1-PH DNA for the Generation of Recombinant AcMNPV

In light of the different behaviour of polyhedrin minus AcMNPV derivatives in which the sKK-0 gene is expressed from the polyhedrin promoter (Examples 4,5 & 6) as compared to polyhedrin plus AcMNPV derivatives in which the sKK-0 gene was expressed from the p10 promoter (Examples 7, 8 & 9), we decided to construct a polyhedrin plus derivative in which the sKK-0 gene was expressed from the polyhedrin promoter to establish if improved insecticidal efficacy could be achieved with the polyhedrin promoter/sKK-0 expression unit but not with the p10 promoter/sKK-0 expression unit.

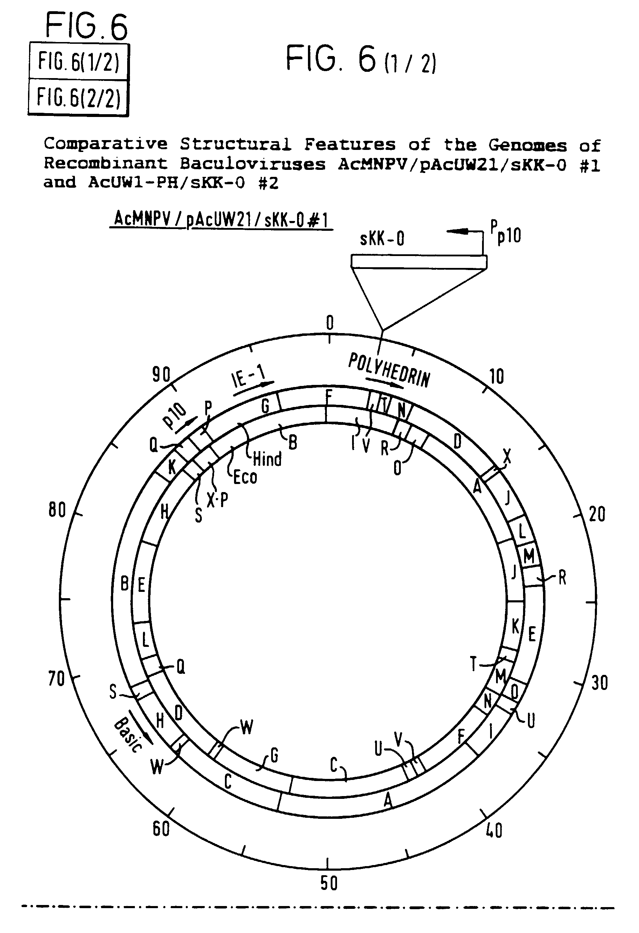

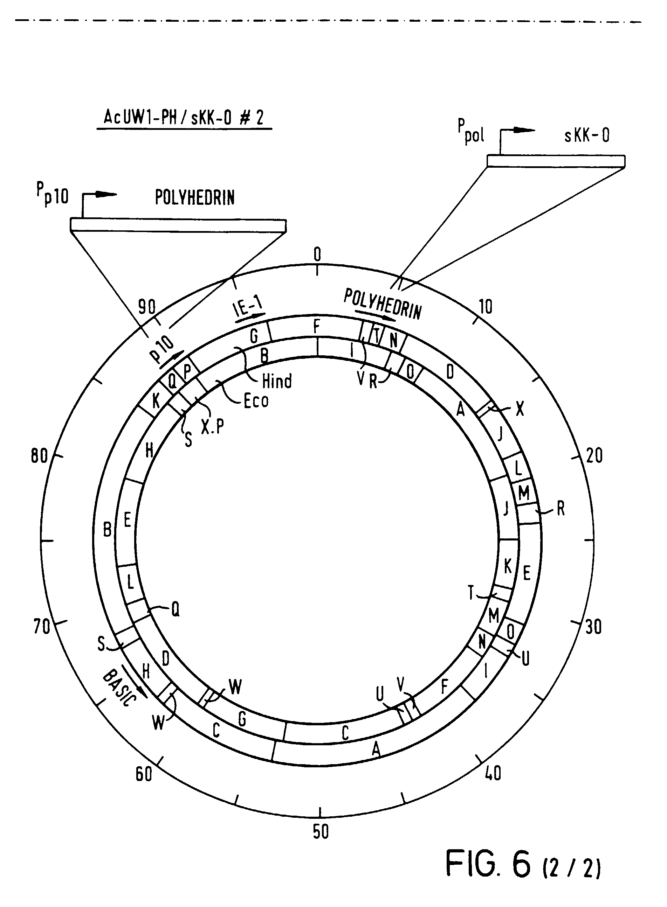

The virus selected as a recipient for the polyhedrin promoter/sKK-0 expression unit was AcUW1-PH (see Weyer et al. (1990) J.Gen.Virol. **71** 1525-1534 and Figure 6). This virus carries a p10 promoter/polyhedrin gene expression unit at the locus occupied by the p10 gene in wild type AcMNPV and a polyhedrin promoter/lacZ indicator gene expression unit at the location normally occupied by the polyhedrin gene. It will therefore form plaques on an Sf21 cell monolayer which contain cells carrying AcMNPV occlusion bodies and which stain blue in the presence of X-Gal indicator (GIBCO/BRL, Grand Island, New York). Replacement of the polyhedrin promoter/lacZ expression unit with the polyhedrin promoter/sKK-0 expression unit of the pVL1392/sKK-0 #2.2 recombinant transfer vector was therefore anticipated to be a convenient means of preparing a polyhedrin plus AcMNPV derivative carrying a polyhedrin promoter/sKK-0 gene expression unit.

A stock of of AcUW1-PH virus of unknown titre was kindly provided by Dr R. D. Possee (NERC Institute of Virology and Environmental Microbiology, Mansfield Road, Oxford). Small scale amplification cultures of this virus were then prepared by seeding 3 lots of $1.5 \times 10^6$ Sf21 cells in 4 ml. TC100/10% foetal calf serum medium (both from GIBCO/BRL) in 25 cm$^2$ tissue culture flasks (Bibby, Stone, Staffs.), incubation overnight at 28°C, followed by addition of 50 µl, 5 µl and 5 µl of a 1/10 dilution aliquots of the stock virus to one flask each and continuing incubation at 28°C for a further 6 days. These small scale amplifications were titered using the standard plaque assay technique (King & Possee (1992) "The Baculovirus Expression System: A Laboratory Guide" (Chapman & Hall)). All plaques obtained contained inclusion bodies and stained blue in the presence of X-Gal. The virus stock produced from the initial 50µl input culture had a titre of $2.3 \times 10^7$ pfu/ml and was used to produce larger, 200 ml. spinner stock culturess of AcUW1-PH, from which purified stocks of virus and subsequently viral DNA were prepared by standard methods (King & Possee (1992) ibid. & Possee & Howard (1987) Nucleic Acids Research **15** 10233-10248). A stock of linearised AcUW1-PH DNA was then prepared by digestion of 3µg viral DNA with SauI (Boehringer Mannheim) according to the manufacturer's recommendations since this enzyme cleaves only within the β galactosidase gene and, as with AcMNPV.lacZ, this was anticipated to be a procedure which would facilitate production and detection of recombinant AcMNPV progeny (King & Possee (1992) ibid.).

## EXAMPLE 11

### Generation of Recombinant (Polyhedrin Plus) AcMNPV Carrying a Polyhedrin Promoter/sKK-0 Gene Expression Unit

Using standard baculovirus techniques (King & Possee (1992) ibid.), recombinant AcMNPV viruses carrying sKK-0 under the transcriptional control of the polyhedrin promoter, with an active polyhedrin gene, were then generated by using the SauI linearised AcUW1-PH virus DNA as a recipient for the polyhedrin promoter/sKK-0 expression module from pVL1392/sKK-0 #2.2. This was accomplished by co-transfection of Sf21 cells with 200 ng. SauI linearised AcUW1-PH DNA prepared as described in Example 10 and 1 μg pVL1392/sKK-0 #2.2 DNA. Using the plaque assay technique, where the viruses generate plaques in Sf21 cell monolayer culture, recombinant virus were selected by initially screening for viruses which lacked the ability to metabolise X-gal (GIBCO/BRL, Grand Island, New York) but retained the ability to produce occlusion bodies (polyhedra). Six such lacZ- virus (AcUW1-PH/KK0 #1, AcUW1-PH/KK0 #2, ACUW1-PH/KK0 #3, ACUW1-PH/KK0 #4, AcUW1-PH/KK0 #5, AcUW1-PH/KK0 #6) were picked. A second plaque assay was then performed with each of the above six clones on Sf21 to purify them to homogeneity and confirm their inability to synthesise βgalactosidase and their ability to produce polyhedra. Small scale cultures of each purified virus were then prepared by the methods outlined in Example 5. Aliquots of each of these virus stocks were then taken for bioassay (see Example 12). In parallel aliquots of the small scale virus stocks were used to prepare small samples of viral DNA for physical analysis.

Southern blot analysis were performed on the prospective AcUW1-PH/pVL1392/sKK-0 viral DNAs. These DNAs were treated with BglII and BscI (an isoschizomer of ClaI), fractionated by electrophoresis on a 1.4% agarose gel, transferred to Hybond-N filters and hybridised with an isolated, random-primed $\alpha^{32}$P-dCTP labelled, sKK-0 probe. These studies confirmed that viruses AcUW1-PH/sKK-0 #2, AcUW1-PH/sKK-0 #4, AcUW1-PH/sKK-0 #5 and AcUW1-PH/sKK-0 #6 each contained an sKK-0 gene, with all the anticipated physical features, lacked a βgalactosidase gene and produced polyhedrin+ virus particles.

Virus AcUW1-PH/sKK0 #2 was selected for subsequent studies.

## EXAMPLE 12

### Bioassay of Prospective AcUW1-PH/pVL1392/SKK-0 Recombinant Virus

The biological efficacy of putative AcUW1-PH/pVL1392/sKK-0 recombinant viruses was evaluated in a series of injection and oral dosing studies against Heliothis virescens larvae. Injection assays with purified non-occluded virus stocks were undertaken using the methods described in Example 6. Oral dosing studies with purified polyhedra were carried out using a modified version of the droplet feeding assay described in Example 9.

Preliminary injection studies comparing the biological activity of the six putative AcUW1-PH/pVL1392/sKK-0 viruses generated as described in Example 11 indicated that only viruses AcUW1-PH/sKK-0 #2, AcUW1-PH/sKK-0 #4, AcUW1-PH/sKK-0 #5 and AcUW1-PH/sKK-0 #6 might have an enhanced insecticidal effect compared to the wild type virus. AcUW1-PH/pVL1392/sKK-0 #2 was selected for scale-up and further characterisation.

Injection assay data indicated that larvae treated with the AcUW1-PH/pVL1392/sKK-0 #2 virus demonstrated abnormal symptomology from 72 HAT onwards (Table VI). By 96 HAT the majority of larvae were dead and the only survivor was paralysed. All larvae were dead by 144 HAT. In contrast, no abnormal effects were observed in larvae treated with wild type AcMNPV until 120 HAT when 50% of larvae were dead; 100% mortality was recorded for the wild type AcMNPV at 144 HAT.

A series of oral dosing studies was carried out to compare the biological efficacy of AcUW1-PH/pVL1392/sKK-0 and the wild type AcMNPV using purified polyhedra. In all cases, the AcUW1-PH/pVL1392/sKK-0 #2 was shown to have a significantly faster speed of kill than the wild type and hence an improved insecticidal effect. Larvae treated with the AcUW1-PH/pVL1392/sKK-0 virus started to show symptoms of paralysis and death from 72 HAT onwards (Table VII). Probit analysis of the dose response data confirmed that the speed of kill of AcUW1-PH/pVL1392/sKK-0 #2 was significantly faster than that of the wild type AcMNPV at 72 and 96 HAT but that by 120 HAT the wild type virus had caught up.

## EXAMPLE 13

### Construction and Cloning of a Synthetic Gene Encoding the KK-1 Conotoxin

As reported by Woodward et al.(1990) EMBO J. **9** 1015-1020, the cone snail Conus textile produces a series of low molecular weight neuroactive peptides designated as belonging to the "King Kong" toxin family and, as described in Example 1 chemically synthetised preparations of both KK-0 and KK-1 showed significant effects on injection into a panel of pest insect species. Given the further evidence, resulting from the studies described in Example 2, 3, 4, 5, 6, 10, 11 and 12, that AcMNPV derivatives which have the capacity to express a synthetic KK-0 gene have substantially improved insecticidal activity, we have also investigated the capacity of KK-1 to influence the behaviour of recombinant AcMNPV viruses.

The sequence of KK-1 toxin mRNA was described by Woodward et al.(ibid.) alongside that of KK-0 mRNA as a result of cDNA cloning and characterisation studies on Conus textile venom gland preparations. These studies revealed that, although mature KK-0 and and the predicted mature KK-1 have only about 26% identity (see Table 2), they are processed from probable pre-pro-toxins with substantially higher levels of homology and which obviously represent members of a protein family. Specifically KK-1 mRNA codes for a propeptide of 77 amino acids with a high level of sequence homology (88 - 92 %) with KK-0 (78 amino acids) in the region encoding the N-terminal 51 aminoacids, as compared to the level of only 26% in the C-terminal region corresponding to the mature toxins (see Figure 7). Within the presumed signal sequence region (residues 1-22 (approx) according to the rules developed by von Heijne (1986) Nucleic Acids Research 14 4683-4690) amino acid identity is 100%.

In planning a design for a synthetic KK-1 gene (sKK-1), given the absolute identity of the presumed signal sequences for pre-pro-KK-0 and pre-pro-KK-1 we chose only to construct a synthetic DNA fragment encoding the pro-toxin part of KK-1.

More specifically we designed a DNA fragment encoding this region which was flanked by the PstI and XmaIII restriction sites which had been built into the original sKK-0 gene for such gene manipulation purposes (see Figure 2). These features were designed to allow:-

Reduction to a minimum of the amount of the new synthetic KK-1 (sKK-1) gene which we needed to assemble by harnessing the identical, and apparently effective, signal sequence coding part of existing KK-0 constructs

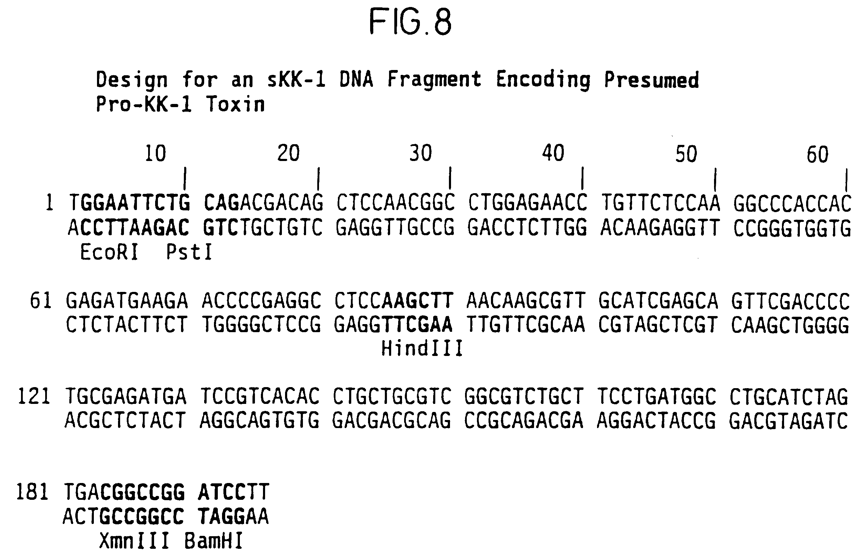

The basic design developed for the sKK-1 gene fragment is outlined in Figure 8.

Additional features of the design for this DNA fragment include:-

* Selection of insect preferred/good codon usage based on data published by Wada et al. (1992) (Nucleic Acids Research 20 Supplement 2111-2118).
* Inclusion of additional flanking EcoRI and BamHI restriction sites to facilitate primary cloning, characterisation and subsequent manipulations of the fragment.

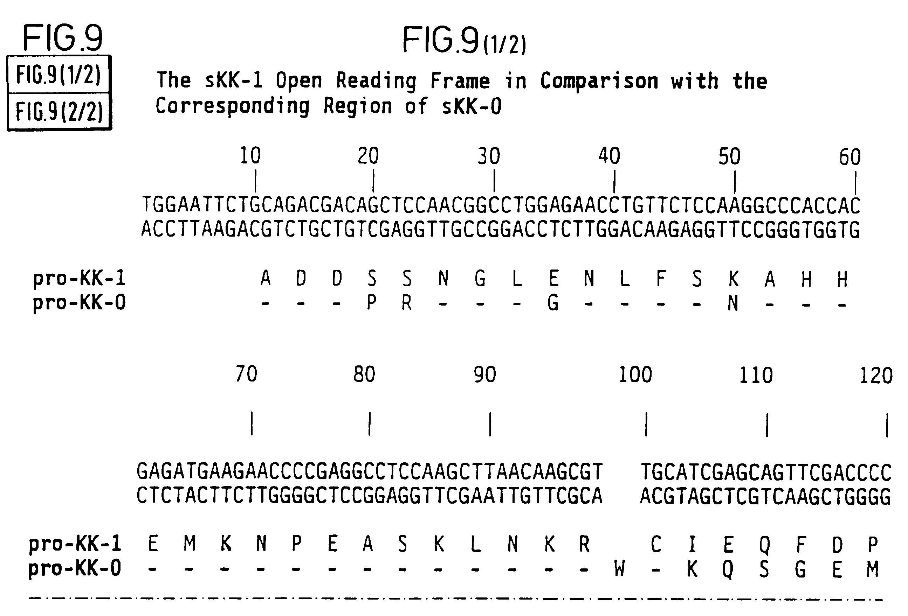

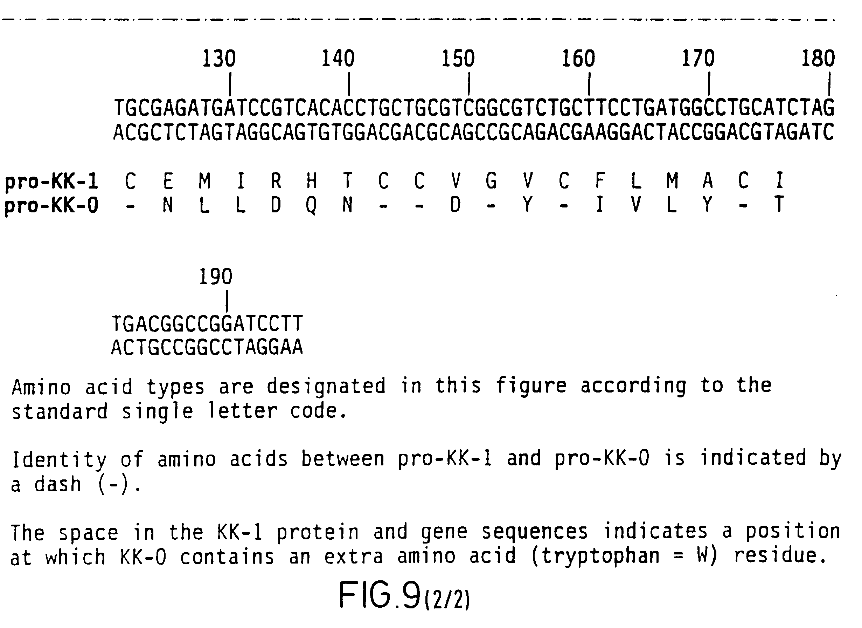

In Figure 9 we show the sequence [SEQ 1D 23] of the novel synthetic sKK-1 fragment, together with amino acids encoded by the major open reading frame (pro-KK-1) [SEQ 1D 24] in comparison with the corresponding sequence of pro KK-0 [SEQ 1D 25].

To assemble the novel sKK-1 pro-toxin gene discussed above and shown in Figure 8, we chose to use an adaptation of the method previously employed successfully to construct the sKK-0 gene.

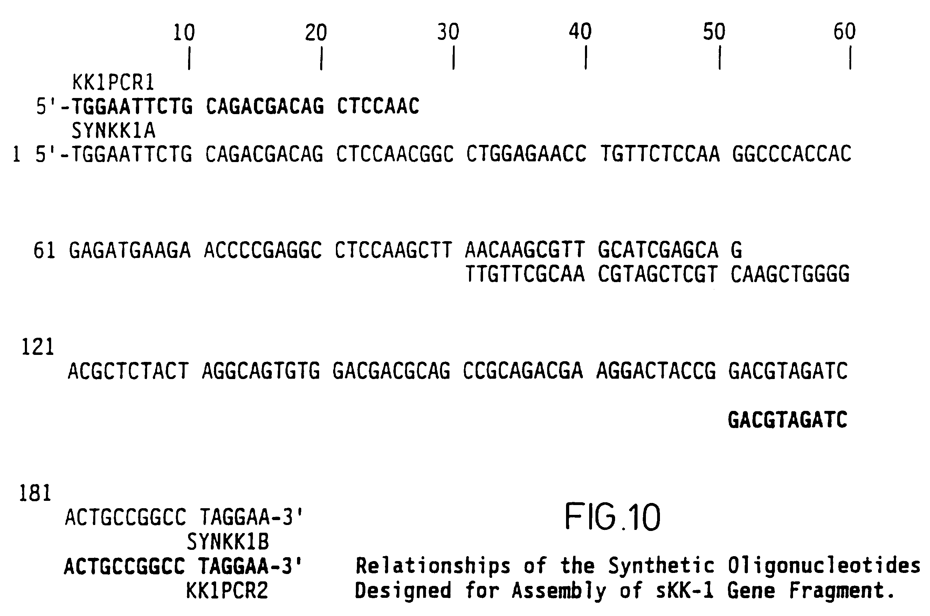

Thus, as shown in Figure 10, two synthetic oligonucleotides - SYNKK1A ( a 111-mer) [SEQ 1D 26] and SYNKK1B (a 106-mer) [SEQ 1D 27], which encode complementary DNA strands and together cover the complete 196nt of sKK-1 with a 21 bp overlap, were synthesised. In addition, two further oligonucleotides (KK1PCR1 and KK1PCR2) [SEQ 1D 28, 29] were prepared, which would be capable of acting as PCR primers to allow amplification of the complete sKK-1 fragment following annealing of SYNKK1A and SYNKK1B and fill in repair with Taq DNA polymerase.

Following synthesis, deprotection etc. of these oligonucleotides as described in Example 3 they were used in a similar manner to that previously described for the sKK-0 gene fragment. Since the sKK-1 fragment was, however, being assembled from only two (rather than four) overlapping oligonucleotides on this occasion only a single "5-cycle PCR repair" reaction was undertaken before progressing to the 25 cycle amplification phase with 0.5μl or 5μl innocula of the repair reaction products.

As previously with the sKK-0 gene, these reactions and suitable controls proceeded exactly as anticipated. Substantial quantities (several μg) of a novel approx. 200bp DNA fragment were generated when all necessary oligonucleotides were included. Omission of individual oligonucleotides eliminated production of this fragment.

Prior to attempting to clone the novel sKK-1 DNA fragment it was worked up by phenol extraction and ethanol precipitation by standard methods (Sambrook et al. (1989) in "Molecular Cloning: A Laboratory Manual" (Cold Spring Harbor Press)).

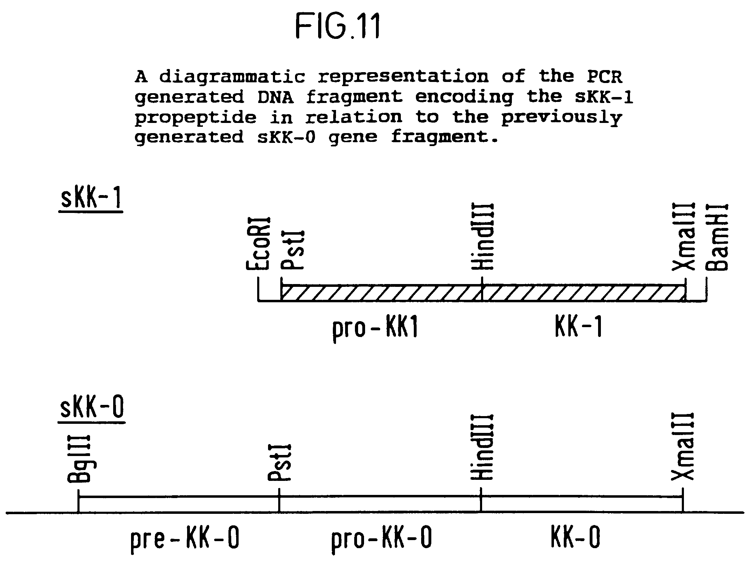

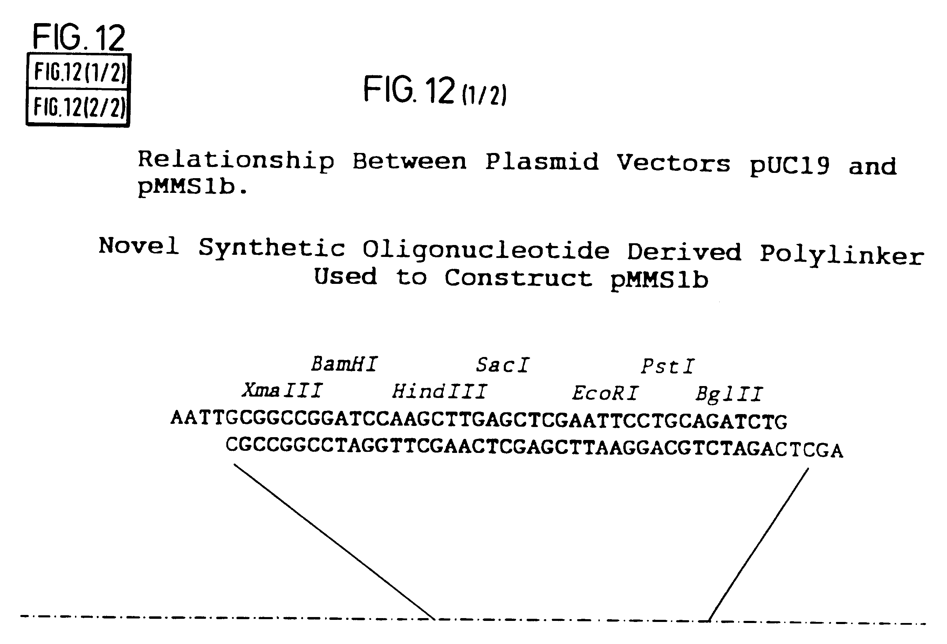

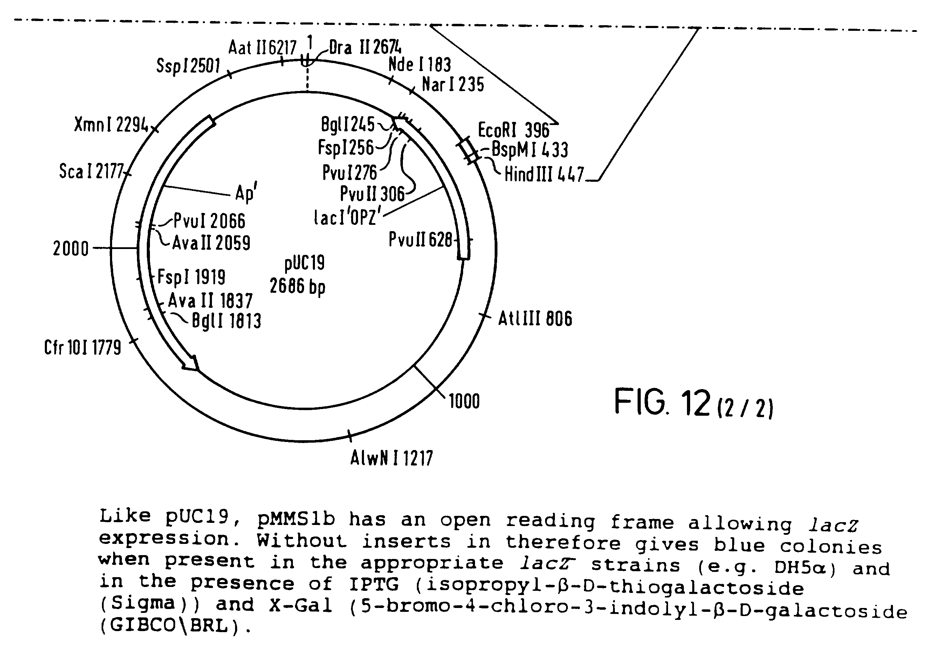

The sKK-1 gene fragment thus prepared should only encode the mature and the presumed pro-sequences but not the signal peptide of the natural KK-1 gene. To verify its structure and obtain material to work with further, it was initially digested with EcoRI and BamHI restriction enzymes (see Figure 8 and 11). It was then cloned into the EcoRI and BamHI sites of pMMS1B - a simple derivative of pUC19 carrying an alternative polylinker sequence [SEQ 1D 30] which rendered it particularly convenient for accomodating and manipulating the sKK-1 fragment (see Figure 12). The products of this ligation were transformed into competent E.coli DH5α cells and twelve putative recombinants were picked for further analysis. The above clones were analysed by restriction enzyme analysis using the cloning enzymes to release the insert. Six of the clones, containing an apparently correctly sized insert, were then chosen for sequence analysis essentially as described in Example 4 using the so-called universal and reverse M13 sequencing primers which recognise regions flanking the polylinker region of this pUC19 derivative. One of the six recombinants (pMMS/KK-1 #3.2) had an insert which matched precisely that expected for sKK-1 cloned in the anticipated manner. This clone was used for the final assembly of the complete sKK-1 gene by fusion to the pro-peptide coding sequence of sKK-0 as follows.

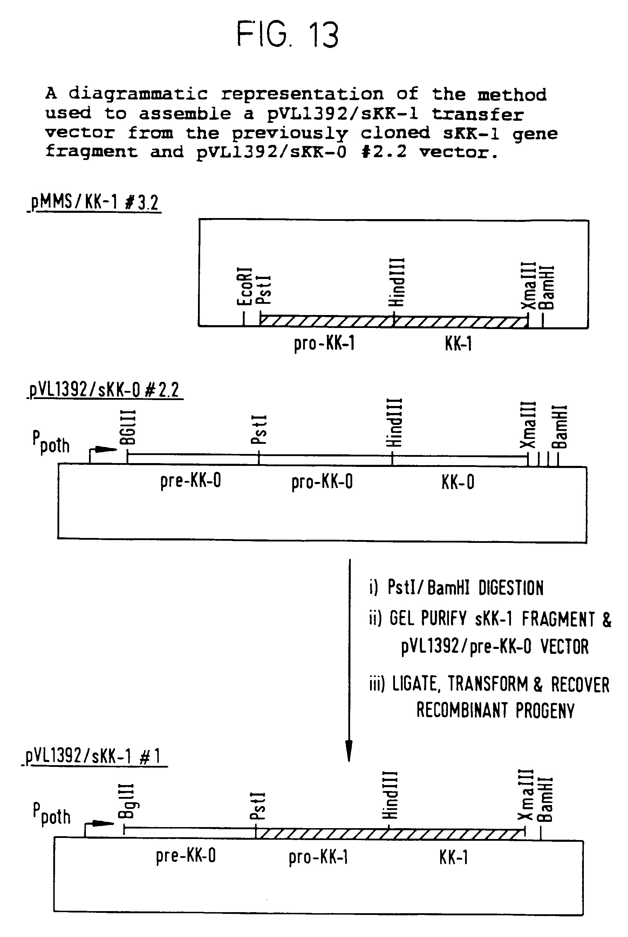

The PstI/BamHI fragment of clone pMMS/KK-1 #3.2 was isolated and subcloned into pVL1392/sKK-0 #2.2 vector which had been digested with PstI/BamHI according to the manufacturers rcommendations (see Figure 13) to provide a gene fragment encoding a complete pre-pro KK-1 primary transcript - comprising the signal sequence of sKK-0 and the pro and mature sequences of sKK-1 - in the pVL1392 transfer vector. The ligation products were then transformed into competent E.coli DH5α cells and twelve putative recombinants were picked for further analysis. The above clones were analysed by restriction enzyme cleavage using the cloning enzymes and EcoRI which should only cut the pVL1392/sKK-0 clone and not the pVl1392/sKK-1 recombinants. Based on this analysis two of the clones were then chosen for sequence analysis. One of these recombinants (pVL1392/sKK-1 #1) had the anticipated sequence and was chosen for recombinant AcMNPV assembly.

## EXAMPLE 14

### Generation of Recombinant (Polyhedrin Minus) AcMNPV Carrying a Polyhedrin Promoter/sKK-1 Gene Expression Unit

The methods utilised here were identical to those described in Example 4.

Therefore, using the methods described in Example 4, six putative AcMNPV/pVL1392/sKK-1 (isolates 1 to 6) recombinant viruses were prepared. Physical analyses were then undertaken to establish which viruses carried intact sKK-1 genes. As previously described, biological assays were performed on all 6 viruses.

The results were:

. Three of the six possible pVL1392/sKK-1 (1, 5 and 6) isolates had a complete sKK-1 gene.

. None of these viruses displayed any enhancement in insecticidal activity when injected into H.virescens larvae.

EP 0 690 920 B1

**Table 1 : Sequence Similarities Between Biologically Active Peptides in the ω-Conotoxin/"King-Kong" Conotoxin Family**

```
Cono-KK-0      WCKQSGEMCNLLDQN--CCDG-YCIVLV-------CT
Cono-KK-1       CIEQFDPCEMIRHT--CCVGV-CFLMA-------CI
Cono-KK-2       CAPFLHPCTFFFPN--CCN-SYCVQFI-------CL
Cono-GVIA       CKSPGSSCSPTSYN--CCR-S-CNPYTK-----RCY
Cono-MVIIB      CKGKGASCHRTSYD--CCTGS-CNRG-K------C
μ-Agatoxin 3   ADCVGDGQRCAD-WAGPYCCSGYYCSCR--SMPYCRCRSDS
Curtatoxin 2   ADCVGDGQKCAD-WFGPYCCSGYYCSCR--SMPYCRCRSDS
Buthus Pep 2   VGCEEDPMNCKGKQAKPTCCNGV-CN---------C-NV
CTL             ACAETGAVCVHNDE---CCSGA-CSPIF---NY--CLPQ
Mj-AMP1         QCIGNGGRCNENVGPPYCCSGF-CL-RQPGQGYGYCKNR
```

| | KK-0 | KK-1 | KK-2 | GVIA | MVIIB | μ-Aga3 | Curt 2 | BuPep2 | CTL | Mj-AMP1 |
|---|---|---|---|---|---|---|---|---|---|---|
| KK-0 | - | 7/27 | 8/27 | 9/27 | 9/27 | 9/27 | 9/27 | 7/27 | 9/27 | 9/27 |
| KK-1 | 7/26 | - | 7/26 | 6/26 | 7/26 | 7/26 | 7/26 | 9/26 | 8/26 | 8/26 |
| KK-2 | 8/26 | 7/26 | - | 8/26 | 7/26 | 7/26 | 7/26 | 7/26 | 7/26 | 6/26 |
| GVIA | 9/27 | 6/27 | 8/27 | - | 15/27 | 8/27 | 8/27 | 7/27 | 8/27 | 7/27 |
| MVIIB | 9/25 | 7/25 | 7/25 | 15/25 | - | 9/25 | 9/25 | 8/25 | 9/25 | 10/25 |
| μAgA 3 | 9/38 | 7/38 | 7/38 | 8/38 | 9/38 | - | 36/38 | 10/38 | 9/38 | 14/38 |
| Curt 2 | 9/38 | 7/38 | 7/38 | 8/38 | 9/38 | 36/38 | - | 9/38 | 11/38 | 14/38 |
| BuPep2 | 7/28 | 9/28 | 7/28 | 7/28 | 8/28 | 10/28 | 9/28 | - | 8/28 | 9/28 |
| CTL | 9/30 | 8/30 | 7/30 | 8/30 | 9/30 | 9/30 | 11/30 | 8/30 | - | 11/30 |
| Mj-AMP1 | 9/37 | 8/37 | 6/37 | 7/37 | 10/37 | 14/37 | 14/37 | 9/37 | 11/37 | - |

## Table 2 : Percentage Similarities Between Biologically Active Peptides in the ω-Conotoxin/"King-Kong" Conotoxin Family

```
Cono-KK-0        WCKQSGEMCNLLDQN--CCDG-YCIVLV-------CT
Cono-KK-1         CIEQFDPCEMIRHT--CCVGV-CFLMA-------CI
Cono-KK-2         CAPFLHPCTFFFPN--CCN-SYCVQFI-------CL
Cono-GVIA         CKSPGSSCSPTSYN--CCR-S-CNPYTK-----RCY
Cono-MVIIB        CKGKGASCHRTSYD--CCTGS-CNRG-K------C
μ-Agatoxin 3     ADCVGDGQRCAD-WAGPYCCSGYYCSCR--SMPYCRCRSDS
Curtatoxin 2     ADCVGDGQKCAD-WFGPYCCSGYYCSCR--SMPYCRCRSDS
Buthus Pep 2     VGCEEDPMNCKGKQAKPTCCNGV-CN---------C-NV
CTL              ACAETGAVCVHNDE---CCSGA-CSPIF---NY--CLPQ
Mj-AMP1          QCIGNGGRCNENVGPPYCCSGF-CL-RQPGQGYGYCKNR
```

| | KK-0 | KK-1 | KK-2 | GVIA | MVIIB | μ-Aga3 | Curt 2 | BuPep2 | CTL | Mj-AMP1 |
|---|---|---|---|---|---|---|---|---|---|---|
| KK-0 | - | 26% | 30% | 33% | 33% | 33% | 33% | 26% | 33% | 33% |
| KK-1 | 27% | - | 27% | 23% | 27% | 27% | 27% | 35% | 31% | 31% |
| KK-2 | 31% | 27% | - | 31% | 27% | 27% | 27% | 27% | 27% | 23% |
| GVIA | 33% | 22% | 30% | - | 56% | 30% | 30% | 26% | 30% | 26% |
| MVIIB | 36% | 28% | 28% | 60% | - | 36% | 36% | 32% | 36% | 40% |
| μAgA 3 | 24% | 18% | 18% | 21% | 24% | - | 95% | 26% | 24% | 37% |
| Curt 2 | 24% | 18% | 18% | 21% | 24% | 95% | - | 24% | 29% | 37% |
| BuPep2 | 25% | 32% | 25% | 25% | 29% | 36% | 32% | - | 29% | 32% |
| CTL | 30% | 27% | 23% | 27% | 30% | 30% | 37% | 27% | - | 37% |
| Mj-AMP1 | 24% | 22% | 16% | 19% | 27% | 38% | 38% | 24% | 30% | - |

EP 0 690 920 B1

Table III:

| Preliminary assessment of the biological activity of synthetic preparations of KK-0, KK-1 AND KK-2 proteins by injection into <u>Heliothis virescens</u> larvae[1] | | |
|---|---|---|
| Treatment | Dose per insect | Effects |
| KK-0 | 1 μl | Loss of coordination within 30 secs post-injection. All larvae showing flaccid paralysis within 2-4 mins of injection & unable to stand, reinvert or respond to stimulation; limp & pliable. Effects began to wear off after 4-6 minutes & most larvae appeared normal at 10 mins post-injection. All larvae alive & well at 24 HAT. |
| | 2 μl | Symptoms as above but more severe & prolonged. Gradual recovery with all larvae appearing normal at 45 mins after injection. All alive & well at 24 HAT. |
| KK-1 | 1 μl | Symptoms first observed within 30 secs of injection. Most larvae showing flaccid paralysis within 2-4 minutes: symptoms as for KK-0. Effects were short-lived & all larvae appeared normal at 6-10 mins post-injection. All larave alive & normal at 24 HAT. |
| KK-2 | 1 μl | No adverse effects observed even at 24 HAT. |
| Control[2] | - | No adverse effects observed during experiment. |

[1] Six fifth-instar larvae injected per treatment; average body mass: 300 mg

[2] Control: 2 μl 0.1M ammonium bicarbonate

Table IV:

| Preliminary assessment of the biological activity of synthetic preparations of KK-0, KK-1 and KK-2 proteins against <u>Periplaneta americana</u>[1] by injection. | | |
|---|---|---|
| Treatment | Dose per insect | Effects |
| KK-0 | 1 μl | Temporary paralysis of back legs observed within 1 min post-injection but effects had disappeared by 6 mins pi. Capable of responding normally to stimulation but locomotion slow & poorly coordinated from 20 min-3h pi. Moribund by 21 HAT & dead at 22HAT. |
| | 5 μl | Tremoring followed by paralysis of back legs within 30 secs pi. Knocked down with mid- & hind legs paralysed by 20 min pi but front legs still working normally. Complete paralysis within 1 HAT. No recovery - moribund at 21 HAT & dead at 22 HAT. |
| | 10 μl | Tremoring & loss of coordination initially in back legs but rapidly spreading to mid- & front legs followed by total paralysis. No recovery - moribund/dead at 22 HAT. |
| KK-1 | 1 μl | Tremors followed by loss of movement in hind legs within 15 sec pi; front legs still actively moving at 5 min pi. Moribund at 20 min pi. Other symptoms included back arching. Some recovery between 1.5-3 HAT. At 21 HAT, insects knocked down & unable to reinvert. No recovery by 52 HAT. |
| | 5 μl | Loss of coordination & paralysis in hind legs immediately after injection, rapidly spreading to mid- & front legs. Complete paralysis within 5 min pi. No recovery & insects dead at 21 HAT. |
| | 10 μl | Rapid loss of coordination followed by complete paralysis within 30 secs pi. Occasional tremors observed thereafter but insects remained knocked down for remainder of experiment. No recovery & all dead at 21HAT. |

[1] Adult male roaches with an average body mass of 840 mg

Table IV:   (continued)

| Preliminary assessment of the biological activity of synthetic preparations of KK-0, KK-1 and KK-2 proteins against <u>Periplaneta americana</u>[1] by injection. | | |
|---|---|---|
| Treatment | Dose per insect | Effects |
| KK-2 | 1 μl | No abnormal effects observed throughout experiment. |
| | 2 μl | Abnormal symptomology included arching back & poorly coordinated locomotion within 1 min pi. Some recovery observed from 20 min pi onwards but insects dead at 69 HAT. |
| | 5 μl | Temporary loss of coordination immediately after injection; other symptoms included dorsal arching. Insects appeared to have recovered by 1.5 HAT but were dead at 45 HAT. |
| Control[2] | - | No abnormal effects observed throughout experiment. |

[1] Adult male roaches with an average body mass of 840 mg

[2] Control: 10 μl 0.1M ammonium bicarbonate solution

Table V: Evaluation of the biological efficacy of AcMNPV/pVL1392/sKK-0 CONO C by injection into <u>Heliothis virescens</u> larvae[6]

| Treatment[1] | Number dead (+ affected) | | | | | |
|---|---|---|---|---|---|---|
| | 24 hr | 48 hr | 72 hr | 96 hr | 120 hr | 144 HR |
| AcMNPV/pVL1392/ lacZ[1] | 0/10 | 0/10 | 0/10 | 0/10 | 1/10 | 6/10 |
| AcMNPV/pVL1392/ sKK-0 Cono C[1] | 0/11 | 0/11 | 0/11 (+3FP[3],1PP[4]) | 3/11 (+1FP,3PP,4AF[5]) | 5/11 (+5FP,1PP) | 11/11 |
| Control[2] | 0/7 | 0/7 | 0/7 | 0/7 | 0/7 | 1/7 |

[1] Dose rate = $1.5 \times 10^4$ pfu/larva.
[2] Control = Sterile water.
[3] FP - Full flaccid paralysis: insect is moribund, cannot stand, walk or reinvert & is capable only of limited feeble movements of mouthparts and claspers in response to stimulation; limp & pliable.
[4] PP - Partial paralysis: mid/posterior part of body is partially paralysed; larva is still capable of some locomotory activity and can reinvert but movements are poorly coordinated.
[5] AF - Affected: larva responds slowly to stimulation but is unable/unwilling to walk or feed.
[6] Fourth-instar larvae; average body mass = 100 mg.

EP 0 690 920 B1

Table VI: Evaluation of the biological efficacy of AcUW1-PH/pVL1392/
sKK-0 #2 and AcMNPV wild type viruses by injection into <u>Heliothis virescens</u> larvae[6].

| Treatment[1] | Number of insects dead (+ affected) | | | | | | |
|---|---|---|---|---|---|---|---|
| | 1 | 2 | 3 | 4 | 5 | 6 | 7 |
| AcMNPV wt | 0/6 | 0/6 | 0/6 | 0/6 | 3/6 | 6/6 | 6/6 |
| AcUW1-PH/ pVL1392/KK0-2 | 0/6 | 0/6 | 0/6 (+1PP[4],1AF[5]) | 5/6 (+1FP[3]) | 6/6 | 6/6 | 6/6 |
| AcUW1-PH/ pVL1392/lacZ | 0/7 | 0/7 | 0/7 | 0/7 | 0/7 | 3/7 | 7/7 |
| Control[2] | 0/5 | 0/5 | 0/5 | 0/5 | 0/5 | 0/5 | 0/5 |

[1] Dose = ~ $1 \times 10^4$ pfu/larva

[2] Control: Sterile water

[3] FP - flaccid paralysis; moribund and cannot stand, reinvert, walk or respond normally to stimulation; only sign of life indicated by very feeble movements of mouthparts/claspers in response to stimulation.

[4] PP - partial paralysis; cannot reinvert or walk; mid- & posterior part of body is completely immobilised but anterior portion of body can still move & pro- and false-legs can still be withdrawn in response to stimulation.

[5] AF - affected; insect can stand, reinvert & respond normally to stimulation but is incapable of coordinated locomotion.

[6] Fifth-instar larvae, average body mass = 300 mg.

EP 0 690 920 B1

Table VII:  Evaluation of the biological efficacy of AcUW1-PH/pVL1392/
sKK-O #2 and AcMNPV wild type viruses against <u>H.virescens</u> larvae by oral
dosing

| Treatment | Dose (PIBs/ml) | % kill (+ % affected[2]) | | | | |
|---|---|---|---|---|---|---|
| | | 72 hr | 96 hr | 120 hr | 144 hr | 168 hr |
| AcMNPV WT | $1.0 \times 10^7$ | 3 | 33 | 67 | 70 | 70 |
| | $3.3 \times 10^6$ | 0 | 7 | 17 | 20 | 20 |
| | $1.1 \times 10^6$ | 0 | 13 | 20 | 20 | 20 |
| | $3.7 \times 10^5$ | 0 | 0 | 7 | 10 | 10 |
| | $1.2 \times 10^5$ | 0 | 0 | 3 | 3 | 3 |
| | $4.1 \times 10^4$ | 0 | 0 | 4 | 4 | 4 |
| AcUW1-PH pVL1392 sKK-O #2 | $1.0 \times 10^7$ | 7 | 37 (+30) | 67 | 73 | 73 |
| | $3.3 \times 10^6$ | 3 | 17 (+10) | 31 | 31 | 34 |
| | $1.1 \times 10^6$ | 4 (4) | 18 (+7) | 32 | 32 | 32 |
| | $3.7 \times 10^5$ | 3 | 10 (+3) | 14 | 14 | 14 |
| | $1.2 \times 10^5$ | 7 (+3) | 10 | 13 (+3) | 17 (3) | 20 |
| | $4.1 \times 10^4$ | 3 | 3 | 3 | 3 | 3 |
| Control | - | 0 | 0 | 0 | 0 | 0 |

| Time after treatment (hr) | AcMNPV wild type | | AcUW1-PH/pVL1392/sKK-0#2 | |
|---|---|---|---|---|
| | $LC_{50}$[3] | 95% C.I. | $LC_{50}$ | 95% C.I. |
| 96 | - | - | $6.1 \times 10^6$ | $3.3 \times 10^6 - 1.7 \times 10^7$ |
| 120 | $7.6 \times 10^6$ | $4.4 \times 10^6 - 1.9 \times 10^7$ | $5.2 \times 10^6$ | $2.7 \times 10^6 - 1.6 \times 10^7$ |
| 144 | $6.6 \times 10^6$ | $3.9 \times 10^6 - 1.5 \times 10^7$ | $4.3 \times 10^6$ | $2.3 \times 10^6 - 1.2 \times 10^7$ |
| 168 | $6.6 \times 10^6$ | $3.9 \times 10^6 - 1.5 \times 10^6$ | $4.0 \times 10^6$ | $2.2 \times 10^6 - 1.0 \times 10^7$ |

[1]  Neonate larvae dosed using a modified version of the droplet feeding
assay (Hughes & Wood, 1981); 30 larvae/dose.
[2]  ( )  % paralysed larvae
[3]  $LC_{50}$ values (PIBs/ml virus suspension) estimated using a standard
logit dose response procedure (Ashton, 1972).

## Claims

1. Synthetic DNA having the sequence shown in Sequence ID No. 1:

```
TTAGATCTAA TTCACCATGA AGCTGACATG TATGATGATC GTGGCCGTGC TGTTCCTGAC      60
CGCCTGGACC TTCGCCACTG CAGACGATCC CCGCAACGGC CTGGGCAACC TGTTCTCCAA     120
CGCCCACCAC GAAATGAAGA ACCCCGAGGC ATCCAAGCTT AACAAGCGCT GGTGTAAGCA     180
GTCCGGAGAG ATGTGTAACC TGCTGGACCA GAACTGTTGT GACGGCTACT GTATCGTGCT     240
GGTGTGCACC TAGTGACGGC CGGATCCTT                                       269.
```

2. Synthetic DNA according to claim 1 having greater than 77% homology with the sequence shown in Sequence ID No. 1:

```
TTAGATCTAA TTCACCATGA AGCTGACATG TATGATGATC GTGGCCGTGC TGTTCCTGAC      60
CGCCTGGACC TTCGCCACTG CAGACGATCC CCGCAACGGC CTGGGCAACC TGTTCTCCAA     120
CGCCCACCAC GAAATGAAGA ACCCCGAGGC ATCCAAGCTT AACAAGCGCT GGTGTAAGCA     180
GTCCGGAGAG ATGTGTAACC TGCTGGACCA GAACTGTTGT GACGGCTACT GTATCGTGCT     240
GGTGTGCACC TAGTGACGGC CGGATCCTT                                       269.
```

3. A method of controlling an insect population at a locus which comprises administering to the insects or locus a peptide protein toxic to insects, said protein being capable of expression from a DNA sequence as claimed in claim 1 or claim 2.

4. A biological control agent comprising a viral, prokaryotic or eukaryotic host organism the genome of which has been modified by incorporation of a DNA sequence as claimed in claim 1 or claim 2.

5. A biological control agent according to claim 4 wherein the host organism is a baculovirus or an entomopoxvirus.

6. A biological control agent according to claim 5 wherein the host organism is a baculovirus and expression of the peptide protein is under the control of the polyhedrin promoter.

7. A biological control agent according to claim 5 wherein the host organism is an baculovirus and expression of the peptide protein is under the control of the p10 promoter.

8. A biological control agent according to claim 5 wherein the host organism is an entomopoxvirus and expression of the peptide protein is under the control of the spheroidin promoter.

9. A biological control agent according to claim 4 wherein the host organism is a bacterial or fungal organism which infects or is in symbiotic association with insects.

10. A biological control agent according to claim 9 wherein the host organism is Bacillus thuringiensis.

11. A biological control agent according to claim 9 wherein the host organism is Beauvaria bassiana.

12. A biological control agent according to claim 9 wherein the host organism is a plant endophyte.

13. A method according to claim 3 wherein the peptide protein is administered to the locus in the form of biological control agent according to claim 4.

14. A method according to claim 13 wherein the biological control agent is a plant endophyte and the locus is a plant or the seed of a plant.

15. A transmission vector comprising a synthetic DNA sequence encoding a gene capable of expressing the protein of Sequence ID. No. 2:

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu


    Asn Lys Arg Trp Cys Lys Gln Ser Gly Glu Met Cys Asn Leu Leu Asp
    Gln Asn Cys Cys Asp Gly Tyr Cys Ile Val Leu Val Cys Thr
```

or a protein having at least 78% homology thereto.

16. A transmission vector according to claim 15 which is a plasmid.

17. A transmission vector according to claim 16 wherein the plasmid is pVL1392 or pAcUW21.

18. A biological control agent according to claim 4 comprising a baculovirus the genome of which is modified by incorporation of a synthetic DNA sequence encoding a gene capable of expressing the protein of Sequence ID. No. 2:

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu
Asn Lys Arg Trp Cys Lys Gln Ser Gly Glu Met Cys Asn Leu Leu Asp
Gln Asn Cys Cys Asp Gly Tyr Cys Ile Val Leu Val Cys Thr
```

or a protein having at least 78% homology thereto.

**19.** A bacterial host organism comprising a plasmid vector according to claim 16.

**20.** A bacterial host organism comprising a plasmid vector according to claim 19 wherein the host is <u>Escherichia</u> <u>coli</u> and the plasmid is PVL1392.

**21.** A bacterial host organism comprising a plasmid vector according to claim 19 having the characteristics of JHCC8002 (NCIMB 40540).

**Patentansprüche**

**1.** Synthetische DNA, die die in Sequenz ID Nr. 1 dargestellte Sequenz hat:

```
TTAGATCTAA TTCACCATGA AGCTGACATG TATGATGATC GTGGCCGTGC TGTTCCTGAC      60
CGCCTGGACC TTCGCCACTG CAGACGATCC CCGCAACGGC CTGGGCAACC TGTTCTCCAA     120
CGCCCACCAC GAAATGAAGA ACCCCGAGGC ATCCAAGCTT AACAAGCGCT GGTGTAAGCA     180
GTCCGGAGAG ATGTGTAACC TGCTGGACCA GAACTGTTGT GACGGCTACT GTATCGTGCT     240
GGTGTGCACC TAGTGACGGC CGGATCCTT                                       269.
```

**2.** Synthetische DNA nach Anspruch 1, die eine Homologie über 77 % mit der in Sequenz ID Nr. 1 dargestellten Sequenz hat:

```
TTAGATCTAA TTCACCATGA AGCTGACATG TATGATGATC GTGGCCGTGC TGTTCCTGAC      60
CGCCTGGACC TTCGCCACTG CAGACGATCC CCGCAACGGC CTGGGCAACC TGTTCTCCAA     120
CGCCCACCAC GAAATGAAGA ACCCCGAGGC ATCCAAGCTT AACAAGCGCT GGTGTAAGCA     180
GTCCGGAGAG ATGTGTAACC TGCTGGACCA GAACTGTTGT GACGGCTACT GTATCGTGCT     240
GGTGTGCACC TAGTGACGGC CGGATCCTT                                       269.
```

**3.** Verfahren zur Bekämpfung einer Insektenpopulation an einer Stelle, das eine Verabreichung eines für Insekten toxischen Peptidproteins an Insekten oder Auftragen desselben an der Stelle umfaßt, wobei das Protein für eine Expression aus einer DNA-Sequenz nach Anspruch 1 oder Anspruch 2 geeignet ist.

**4.** Biologisches Bekämpfungsmittel, das einen prokaryotischen oder eukaryotischen Wirtsorganismus, dessen Genom durch Einbau einer DNA-Sequenz nach Anspruch 1 oder Anspruch 2 modifiziert wurde, enthält.

**5.** Biologisches Bekämpfungsmittel nach Anspruch 4, in dem der Wirtsorganismus ein Baculovirus oder Entomopoxvirus ist.

**6.** Biologisches Bekämpfungsmittel nach Anspruch 5, in dem der Wirtsorganismus ein Baculovirus ist und die Expression des Peptidproteins unter der Kontrolle des Polyhedrinpromotors erfolgt.

**7.** Biologisches Bekämpfungsmittel nach Anspruch 5, in dem der Wirtsorganismus ein Baculovirus ist und die Expression des Peptidproteins unter der Kontrolle des p10-Promotors erfolgt.

**8.** Biologisches Bekämpfungsmittel nach Anspruch 5, in dem der Wirtsorganismus ein Entomopoxvirus ist und die Expression des Peptidproteins unter der Kontrolle des Spheroidin-Promotors erfolgt.

**9.** Biologisches Bekämpfungsmittel nach Anspruch 4, in dem der Wirtsorganismus ein bakterieller oder Pilz-Organismus ist, der Insekten befällt oder in symbiotischer Verbindung mit Insekten steht.

**10.** Biologisches Bekämpfungsmittel nach Anspruch 9, in dem der Wirtsorganismus Bacillus thuringiensis ist.

**11.** Biologisches Bekämpfungsmittel nach Anspruch 9, in dem der Wirtsorganismus Beauvaria bassiana ist.

**12.** Biologisches Bekämpfungsmittel nach Anspruch 9, in dem der Wirtsorganismus ein Pflanzen-Endophyt ist.

**13.** Verfahren nach Anspruch 3, wobei das Peptidprotein in Form eines biologischen Bekämpfungsmittels nach Anspruch 4 auf die Stelle aufgetragen wird.

**14.** Verfahren nach Anspruch 13, wobei das biologische Bekämpfungsmittel ein Pflanzen-Endophyt ist und die Stelle eine Pflanze oder der Samen einer Pflanze ist.

**15.** Transmissionsvektor, der eine synthetische DNA-Sequenz enthält, welche ein Gen kodiert, das fähig ist, das Protein der Sequenz ID Nr. 2:

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu
Asn Lys Arg Trp Cys Lys Gln Ser Gly Glu Met Cys Asn Leu Leu Asp
Gln Asn Cys Cys Asp Gly Tyr Cys Ile Val Leu Val Cys Thr
```

oder ein Protein mit mindestens 78%iger Homologie dazu zu exprimieren.

**16.** Transmissionsvektor nach Anspruch 15, der ein Plasmid ist.

**17.** Transmissionsvektor nach Anspruch 16, in dem das Plasmid pVL1392 oder pAcUW21 ist.

**18.** Biologisches Bekämpfungsmittel nach Anspruch 4, das einen Baculovirus enthält, dessen Genom durch Einbau einer synthetischen DNA modifiziert ist, welche ein Gen kodiert, das fähig ist, das Protein der Sequenz ID Nr. 2:

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu
Asn Lys Arg Trp Cys Lys Gln Ser Gly Glu Met Cys Asn Leu Leu Asp
Gln Asn Cys Cys Asp Gly Tyr Cys Ile Val Leu Val Cys Thr
```

oder ein Protein mit mindestens 78%iger Homologie dazu zu exprimieren.

**19.** Bakterieller Wirtsorganismus, der einen Plasmidvektor nach Anspruch 16 enthält.

**20.** Bakterieller Wirtsorganismus, der einen Plasmidvektor nach Anspruch 19 enthält, wobei der Wirt Escherichia coli ist und das Plasmid PVL1392 ist.

**21.** Bakterieller Wirtsorganismus, der einen Plasmidvektor enthält, nach Anspruch 19, der die Charakteristika JHCC8002 (NCIMB 40540) hat.

**Revendications**

**1.** ADN synthétique ayant la séquence indiquée à la Séquence identifiée par le numéro 1 :

```
TTAGATCTAA TTCACCATGA AGCTGACATG TATGATGATC GTGGCCGTGC TGTTCCTGAC     60
CGCCTGGACC TTCGCCACTG CAGACGATCC CCGCAACGGC CTGGGCAACC TGTTCTCCAA    120
CGCCCACCAC GAAATGAAGA ACCCCGAGGC ATCCAAGCTT AACAAGCGCT GGTGTAAGCA    180
GTCCGGAGAG ATGTGTAACC TGCTGGACCA GAACTGTTGT GACGGCTACT GTATCGTGCT    240
GGTGTGCACC TAGTGACGGC CGGATCCTT                                      269.
```

2. ADN synthétique selon la revendication 1 ayant un degré d'homologie supérieur à 77% à la séquence indiquée à la Séquence identifiée par le numéro 1 :

```
TTAGATCTAA TTCACCATGA AGCTGACATG TATGATGATC GTGGCCGTGC TGTTCCTGAC     60
CGCCTGGACC TTCGCCACTG CAGACGATCC CCGCAACGGC CTGGGCAACC TGTTCTCCAA    120
CGCCCACCAC GAAATGAAGA ACCCCGAGGC ATCCAAGCTT AACAAGCGCT GGTGTAAGCA    180
GTCCGGAGAG ATGTGTAACC TGCTGGACCA GAACTGTTGT GACGGCTACT GTATCGTGCT    240
GGTGTGCACC TAGTGACGGC CGGATCCTT                                      269.
```

3. Procédé de lutte contre une population d'insectes à un site donné qui comprend l'administration aux insectes ou au site d'une protéine peptidique toxique pour les insectes, ladite protéine étant capable d'être exprimée à partir d'une séquence d'ADN selon la revendication 1 ou 2.

4. Agent de lutte biologique comprenant un organisme hôte viral, procaryote ou eucaryote dont le génome a été modifié par incorporation d'une séquence d'ADN selon la revendication 1 ou la revendication 2.

5. Agent de lutte biologique selon la revendication 4, dans lequel l'organisme hôte est un baculovirus ou un entomopoxvirus.

6. Agent de lutte biologique selon la revendication 5, dans lequel l'organisme hôte est un baculovirus et l'expression de la protéine peptidique est sous la dépendance du promoteur de polyhédrine.

7. Agent de lutte biologique selon la revendication 5, dans lequel l'organisme hôte est un baculovirus et l'expression de la protéine peptidique est sous la dépendance du promoteur p10.

8. Agent de lutte biologique selon la revendication 5, dans lequel l'organisme hôte est un entomopoxvirus et l'expression de la protéine peptidique est sous la dépendance du promoteur de sphéroïdine.

9. Agent de lutte biologique selon la revendication 4, dans lequel l'organisme hôte est un organisme bactérien ou fongique qui infecte ou est en association symbiotique avec des insectes.

10. Agent de lutte biologique selon la revendication 9, dans lequel l'organisme hôte est Bacillus thuringiensis.

11. Agent de lutte biologique selon la revendication 9, dans lequel l'organisme hôte est Beauvaria bassiana.

12. Agent de lutte biologique selon la revendication 9, dans lequel l'organisme hôte est une plante endophyte.

13. Procédé selon la revendication 3, dans lequel la protéine peptidique est administrée au site sous forme d'agent de lutte biologique selon la revendication 4.

14. Procédé selon la revendication 13, dans lequel l'agent de lutte biologique est une plante endophyte et le site est une plante ou la semence d'une plante.

15. Vecteur de transmission comprenant une séquence d'ADN synthétique codant pour un gène capable d'exprimer la protéine de la Séquence identifiée par le numéro 2 :

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu
Asn Lys Arg Trp Cys Lys Gln Ser Gly Glu Met Cys Asn Leu Leu Asp
Gln Asn Cys Cys Asp Gly Tyr Cys Ile Val Leu Val Cys Thr
```

ou une protéine ayant un degré d'homologie d'au moins 78% à celle-ci.

16. Vecteur de transmission selon la revendication 15 qui est un plasmide.

17. Vecteur de transmission selon la revendication 16, dans lequel le plasmide est pVL1392 ou pAcUW21.

18. Agent de lutte biologique selon la revendication 4, comprenant un baculovirus dont le génome est modifié par incorporation d'une séquence d'ADN synthétique codant pour un gène capable d'exprimer la protéine de la Séquence identifiée par le numéro 2 :

```
Met Lys Leu Thr Cys Met Met Ile Val Ala Val Leu Phe Leu Thr Ala
Trp Thr Phe Ala Thr Ala Asp Asp Pro Arg Asn Gly Leu Gly Asn Leu
Phe Ser Asn Ala His His Glu Met Lys Asn Pro Glu Ala Ser Lys Leu
Asn Lys Arg Trp Cys Lys Gln Ser Gly Glu Met Cys Asn Leu Leu Asp
Gln Asn Cys Cys Asp Gly Tyr Cys Ile Val Leu Val Cys Thr
```

ou une protéine ayant un degré d'homologie d'au moins 78% à celle-ci.

19. Organisme hôte bactérien comprenant un vecteur plasmidique selon la revendication 16.

20. Organisme hôte bactérien comprenant un vecteur plasmidique selon la revendication 19, dans lequel l'hôte est Escherichia coli et le plasmide est PVL1392.

21. Organisme hôte bactérien comprenant un vecteur plasmidique selon la revendication 19, ayant les caractéristiques de JHCC8002 (NCIMB 40540).

# FIG.1

| FIG.1 (1/2) |
|---|
| FIG.1 (2/2) |

## FIG.1 (1/2)

Sequence of a Synthetic KK-0 Conotoxin (sKK-0) Gene
Showing the Region Encoding Pre-Pro Conotoxin KK-0

EP 0 690 920 B1

```
          10          20          30          40          50          60

                  MetL ysLeuThrCy sMetMetIle ValAlaValL euPheLeuTh
TTAGATCTAA TTCACCATGA AGCTGACATG TATGATGATC GTGGCCGTGC TGTTCCTGAC
AATCTAGATT AAGTGGTACT TCGACTGTAC ATACTACTAG CACCGGCACG ACAAGGACTG


          70          80          90          100         110         120

rAlaTrpThr PheAlaThrA laAspAspPr oArgAsnGly LeuGlyAsnL euPheSerAs
CGCCTGGACC TTCGCCACTG CAGACGATCC CCGCAACGGC CTGGGCAACC TGTTCTCCAA
GCGGACCTGG AAGCGGTGAC GTCTGCTAGG GGCGTTGCCG GACCCGTTGG ACAAGAGGTT
```

```
        130          140          150          160          170          180
                                                             *
nAlaHisHis  GluMetLysA  snProGluAl  aSerLysLeu  AsnLysArgT  rpCysLysGl
CGCCCACCAC  GAAATGAAGA  ACCCCGAGGC  ATCCAAGCTT  AACAAGCGCT  GGTGTAAGCA
GCGGGTGGTG  CTTTACTTCT  TGGGGCTCCG  TAGGTTCGAA  TTGTTCGCGA  CCACATTCGT


        190          200          210          220          230          240

nSerGlyGlu  MetCysAsnL  euLeuAspGl  nAsnCysCys  AspGlyTyrC  ysIleValLe
GTCCGGAGAG  ATGTGTAACC  TGCTGGACCA  GAACTGTTGT  GACGGCTACT  GTATCGTGCT
CAGGCCTCTC  TACACATTGG  ACGACCTGGT  CTTGACAACA  CTGCCGATGA  CATAGCACGA


        250          260          269

uValCysThr  ---
GGTGTGCACC  TAGTGACGGC  CGGATCCTT
CCACACGTGG  ATCACTGCCG  GCCTAGGAA
```

The arrow (*) indicates the presumed processing site used to release mature
conotoxin KK-0 from it's precursor.

## FIG.1(2/2)

EP 0 690 920 B1

FIG. 2

Sequence of a Synthetic KK-0 Conotoxin Gene Showing
the Location of Restriction Sites Introduced to
Facilitate Cloning and Other Manipulations

```
           10         20         30         40         50         60
    TTAGATCTAA TTCACCATGA AGCTGACATG TATGATGATC GTGGCCGTGC TGTTCCTGAC
    AATCTAGATT AAGTGGTACT TCGACTGTAC ATACTACTAG CACCGGCACG ACAAGGACTG
     BglII                          AflIII
           70         80         90        100        110        120
    CGCCTGGACC TTCGCCACTG CAGACGATCC CCGCAACGGC CTGGGCAACC TGTTCTCCAA
    GCGGACCTGG AAGCGGTGAC GTCTGCTAGG GGCGTTGCCG GACCCGTTGG ACAAGAGGTT
                   PstI
          130        140        150        160        170        180
    CGCCCACCAC GAAATGAAGA ACCCCGAGGC ATCCAAGCTT AACAAGCGCT GGTGTAAGCA
    GCGGGTGGTG CTTTACTTCT TGGGGCTCCG TAGGTTCGAA TTGTTCGCGA CCACATTCGT
                             SecI       HindIII    HaeII
          190        200        210        220        230        240
    GTCCGGAGAG ATGTGTAACC TGCTGGACCA GAACTGTTGT GACGGCTACT GTATCGTGCT
    CAGGCCTCTC TACACATTGG ACGACCTGGT CTTGACAACA CTGCCGATGA CATAGCACGA
     BspMII
          250        260        269
    GGTGTGCACC TAGTGACGGC CGGATCCTT
    CCACACGTGG ATCACTGCCG GCCTAGGAA
     HgiAI           XmaIII BamHI
```

1) Sequences highlighted in bold typescript show the positions of
restriction enzyme recognition sequences introduced to facilitate cloning
and subsequent manipulations but without (a) altering the encoded amino
acid sequence or (b) introducing unfavoured codons.

2) Codons underlined show the translation initiation and termination codons
for "preconotoxin" KK-0.

EP 0 690 920 B1

# FIG.3

Sequence and Relationships of Oligonucleotides Used
for the Assembly of a Synthetic KK-0 Conotoxin Gene
sKK-0

```
          10         20         30         40         50         60
 ConoA
5'-TTAGATCTAA TTCACCATGA AGCTGACATG TATGATGATC GTGGCCGTGC TGTTCCTGAC
5'-TTAGATCTAA TTCACCATGA AGCTGACATG
 ConoPCR1


          70         80         90         100        110        120

CGCCTGGACC TTCGCCACTG CAGA
           CCTGG AAGCGGTGAC GTCTGCTAGG GGCGTTGCCG GACCCGTTGG ACAAGAGGTT
           ConoB


          130        140        150        160        170        180
          ConoC
      5'-CAC GAAATGAAGA ACCCCGAGGC ATCCAAGCTT AACAAGCGCT GGTGTAAGCA
GCGGGTGGTG CTTTACTTCT TGGGGCTCCG TAGG-5'


          190        200        210        220        230        240

GTCCGGAGAG ATGTGTAACC TGCTGGACCA GAACTG
                     CATTGG ACGACCTGGT CTTGACAACA CTGCCGATGA CATAGCACGA
                     ConoD


          250        260        269
                            ConoPCR2
     CACGTGG ATCACTGCCG GCCTAGGAA-5'
CCACACGTGG ATCACTGCCG GCCTAGGAA-5'
```

EP 0 690 920 B1

FIG.4

FIG.4 (1/2)

FIG.4 (2/2)

Cloning and Sequencing Strategy for Generation and Characterisation of pVL1392/sKK-0 Recombinant Transfer Vectors

pVL1392          sKK-0          pVL1392

pVL1392FOR          pVL1392REV

*BglII*          *EclXI (XmaIII)*

Bgl II    Pst I    Not I / Xma III    EcoR I    Xba I    Xma I / Sma I / BamH I

pVL1392    5'-GATCAGATCTGCAGCGGCCGCTCCAGAATTCTAGAAGGTACCCGG-3'
           TCTAGACGTCGCCGGCGAGGTCTTAAGATCTTCCATGGGCCCTAG

BamH I    Xma I / Sma I    Xba I    EcoR I    Not I / Xma III    Pst I / Bgl II

pVL1393    5'-GATCCCGGGTACCTTCTAGAATTCCGGAGCGGCCGCTGCAGATCT-3'
           GGCCCATGGAAGATCTTAAGGCCTCGCCGGCGACGTCTAGACTAG

FIG.4 (1/2)

EP 0 690 920 B1

PH—Wild Type

-7        +1
|         |
          Met  Pro  Asp  Tyr
TATAAAT ATG CCG GAT TAT
          +1
          |
pVL 941        5'-TATAAAT<u>AT</u>TCCGGATTATTCATACCGTCCCACCATCGGGCG cggatcc TTTCCT....TAA....AATAAA—3'

+35  V  +177      +736      +1080
|       |          |          |

4.0 ┌──────── BamH I

PH ⟩MCS ... PH

Recombination Sequences

**pVL1392**
**pVL1393**
**BACULOVIRUS TRANSFER**
**VECTORS (Non-Fusion)**
**9.8Kb**

Recombination Sequences

PUC (Amp)

## FIG. 4 (2/2)

Sequence of pVL1392 Sequencing Primers

<u>pVL1392FOR</u>   5'-CTGTTTTCGTAACAG-3'

<u>pvl1392REV</u>   5'-CGGATTTCCTTGAAG-3'

# FIG.5

| |
|---|
| FIG.5(1/2) |
| FIG.5(2/2) |

## FIG.5(1/2)

Sequence Determined for the sKK-O Insert Region of
Transfer Vector pVL1392/sKK-O #2.2

===02-MAR-1994=================================================================PC/GENE=

SEQUENCE OF PVL1392 / SYNTHETIC CONOTOXIN GENE READ WITH SEQUENCING
OLIGO PVL1392REV   (27/3/92)              CLONE 2.2

```
                10          20          30          40          50          60
                 |           |           |           |           |           |
    1 GGCGCGGATC AGATCTAATT CACCATGAAG CTGACATGTA TGATGATCGT GGCCGTGCTG

   61 TTCCTGACCG CCTGGACCTT CGCCACTGCA GACGATCCCC GCAACGGCCT GGGCAACCTG

  121 TTCTCCAACG CCCACCACGA AATGAAGAAC CCCGAGGCAT CCAAGCTTAA CAAGCGCTGG
```

EP 0 690 920 B1

181 TGTAAGCAGT CCGGAGAGAT GTGTAACCTG CTGGACCAGA ACTGTTGTGA CGGCTACTGT

241 ATCGTGCTGG TGTGCACCTA GTGACGGCCG CTCCAGAATT CT

Total number of bases is: 282.
DNA sequence composition:    64 A;    83 C;    76 G;    59 T; 0 OTHER;

Sequence name: CONO22REV

===02-MAR-1994=====================================================PC/GENE=

Sequence tract emboldened corresponds to that anticipated from
the design of gene sKK-0.

Flanking sequences shown in normal script correspond to the
sequence of the appropriate region of pVL1392 (data from
InVitrogen Corp., 3985 Sorrento Valley Boulevard, San Diego,
California).

FIG.5 (2/2)

# FIG.6

FIG. 6(1/2)

FIG. 6(2/2)

# FIG. 6 (1 / 2)

Comparative Structural Features of the Genomes of Recombinant Baculoviruses AcMNPV/pAcUW21/sKK-0 #1 and AcUW1-PH/sKK-0 #2

AcMNPV / pAcUW21 / sKK-0 #1

FIG. 6 (2 / 2)

# FIG.7

Comparisons of the Predicted Amino Acid Sequences of
(A) the Precursors of *Conus textile* KK-0, KK-1 and
KK-2 Peptidess and (B) Predicted Sequences of the
Corresponding Mature Peptides. (Taken from Woodward
*et al.* (1990) EMBO J. 9 1015-1020).

```
KK-0  ----MKLTCMMIVAVLFLTAWTFATADDPRNGLGNLFSNAHHEMKNPEASKLNKRWCKQSGEMCNLLDQNCCDGYCIVLVCT
KK-1                               SS   E   K              - IEQFDP EMIRHT  V V FLMA I
KK-2                         V     SG   E   K         N    - APFLHP TFFFP   NSY VQFI L
```

■ ALL THREE DIFFERENT
▨ Two identical
☐ All three identical

## B

```
KK-0   W C K Q S G E M C N L L D Q N C C D G Y C I V L V C T
KK-1     C I E Q F D P C E M I R H T C C V G V C F L M A C I
KK-2     C A P F L H P C T F F F P N C C N S Y C V Q F I C L

ωMVIIA C K G K G A K C S R L M Y D C C T G S C R S G K C
```

# FIG.8

Design for an sKK-1 DNA Fragment Encoding Presumed
Pro-KK-1 Toxin

```
          10        20        30        40        50        60
          |         |         |         |         |         |
  1 TGGAATTCTG CAGACGACAG CTCCAACGGC CTGGAGAACC TGTTCTCCAA GGCCCACCAC
    ACCTTAAGAC GTCTGCTGTC GAGGTTGCCG GACCTCTTGG ACAAGAGGTT CCGGGTGGTG
    EcoRI   PstI


 61 GAGATGAAGA ACCCCGAGGC CTCCAAGCTT AACAAGCGTT GCATCGAGCA GTTCGACCCC
    CTCTACTTCT TGGGGCTCCG GAGGTTCGAA TTGTTCGCAA CGTAGCTCGT CAAGCTGGGG
                          HindIII


121 TGCGAGATGA TCCGTCACAC CTGCTGCGTC GGCGTCTGCT TCCTGATGGC CTGCATCTAG
    ACGCTCTACT AGGCAGTGTG GACGACGCAG CCGCAGACGA AGGACTACCG GACGTAGATC


181 TGACGGCCGG ATCCTT
    ACTGCCGGCC TAGGAA
       XmnIII BamHI
```

EP 0 690 920 B1

# FIG.9

FIG.9(1/2)

FIG.9(2/2)

## FIG.9(1/2)

The sKK-1 Open Reading Frame in Comparison with the
Corresponding Region of sKK-0

```
            10          20          30          40          50          60
            |           |           |           |           |           |
TGGAATTCTGCAGACGACAGCTCCAACGGCCTGGAGAACCTGTTCTCCAAGGCCCACCAC
ACCTTAAGACGTCTGCTGTCGAGGTTGCCGGACCTCTTGGACAAGAGGTTCCGGGTGGTG
```

```
pro-KK-1          A  D  D  S  S  N  G  L  E  N  L  F  S  K  A  H  H
pro-KK-0          -  -  -  P  R  -  -  -  G  -  -  -  -  N  -  -  -
```

```
            70          80          90          100         110         120


            |           |           |           |           |           |

GAGATGAAGAACCCCGAGGCCTCCAAGCTTAACAAGCGT   TGCATCGAGCAGTTCGACCCC
CTCTACTTCTTGGGGCTCCGGAGGTTCGAATTGTTCGCA   ACGTAGCTCGTCAAGCTGGGG
```

```
pro-KK-1   E  M  K  N  P  E  A  S  K  L  N  K  R     C  I  E  Q  F  D  P
pro-KK-0   -  -  -  -  -  -  -  -  -  -  -  -  -  W  -  K  Q  S  G  E  M
```

EP 0 690 920 B1

```
              130         140         150         160         170         180
               |           |           |           |           |           |
    TGCGAGATGATCCGTCACACCTGCTGCGTCGGCGTCTGCTTCCTGATGGCCTGCATCTAG
    ACGCTCTAGTAGGCAGTGTGGACGACGCAGCCGCAGACGAAGGACTACCGGACGTAGATC

pro-KK-1   C   E   M   I   R   H   T   C   C   V   G   V   C   F   L   M   A   C   I
pro-KK-0   -   N   L   L   D   Q   N   -   -   D   -   Y   -   I   V   L   Y   -   T


              190
               |
    TGACGGCCGGATCCTT
    ACTGCCGGCCTAGGAA
```

Amino acid types are designated in this figure according to the standard single letter code.

Identity of amino acids between pro-KK-1 and pro-KK-0 is indicated by a dash (-).

The space in the KK-1 protein and gene sequences indicates a position at which KK-0 contains an extra amino acid (tryptophan = W) residue.

## FIG.9(2/2)

EP 0 690 920 B1

```
                   10          20          30          40          50          60
                   |           |           |           |           |           |
     KK1PCR1
  5'-TGGAATTCTG CAGACGACAG CTCCAAC
     SYNKK1A
1 5'-TGGAATTCTG CAGACGACAG CTCCAACGGC CTGGAGAACC TGTTCTCCAA GGCCCACCAC


61 GAGATGAAGA ACCCCGAGGC CTCCAAGCTT AACAAGCGTT GCATCGAGCA G
                                        TTGTTCGCAA CGTAGCTCGT CAAGCTGGGG

121
    ACGCTCTACT AGGCAGTGTG GACGACGCAG CCGCAGACGA AGGACTACCG GACGTAGATC

                                                              GACGTAGATC


181
    ACTGCCGGCC TAGGAA-3'
           SYNKK1B
    ACTGCCGGCC TAGGAA-3'
           KK1PCR2
```

FIG.10

Relationships of the Synthetic Oligonucleotides
Designed for Assembly of sKK-1 Gene Fragment.

FIG.11

A diagrammatic representation of the PCR generated DNA fragment encoding the sKK-1 propeptide in relation to the previously generated sKK-0 gene fragment.

# FIG. 12

| FIG.12(1/2) |
|---|
| FIG.12(2/2) |

## FIG. 12 (1/2)

Relationship Between Plasmid Vectors pUC19 and pMMS1b.

Novel Synthetic Oligonucleotide Derived Polylinker
Used to Construct pMMS1b

```
          BamHI          SacI          PstI
     Xma III        HindIII        EcoRI     BglII
AATTGCGGCCGGATCCAAGCTTGAGCTCGAATTCCTGCAGATCTG
    CGCCGGCCTAGGTTCGAACTCGAGCTTAAGGACGTCTAGACTCGA
```

Aat II 6217  Dra II 2674
Ssp I 2501  Nde I 183
Nar I 235

Xmn I 2294  Bgl I 245  EcoRI 396
Fsp I 256  BspM I 433
Sca I 2177  Pvu I 276  Hind III 447
Ap'  Pvu II 306
lac I' OPZ'
Pvu I 2066  Pvu II 628
Ava II 2059

2000  pUC19
2686 bp

Fsp I 1919
Ava II 1837
Bgl I 1813

Cfr 10 I 1779  Atl III 806

1000  FIG. 12 (2 / 2)

AlwN I 1217

Like pUC19, pMMS1b has an open reading frame allowing *lacZ* expression. Without inserts in therefore gives blue colonies when present in the appropriate *lacZ* strains (e.g. DH5α) and in the presence of IPTG (isopropyl-β-D-thiogalactoside (Sigma)) and X-Gal (5-bromo-4-chloro-3-indolyl-β-D-galactoside (GIBCO\BRL).

# FIG. 13

A diagrammatic representation of the method used to assemble a pVL1392/sKK-1 transfer vector from the previously cloned sKK-1 gene fragment and pVL1392/sKK-0 #2.2 vector.

pMMS/KK-1 #3.2

pro-KK-1     KK-1

EcoRI PstI    HindIII    XmaIII BamHI

pVL1392/sKK-0 #2.2

P_poth

BGlII    PstI    HindIII    XmaIII BamHI

pre-KK-0    pro-KK-0    KK-0

i) PstI/BamHI DIGESTION

ii) GEL PURIFY sKK-1 FRAGMENT & pVL1392/pre-KK-0 VECTOR

iii) LIGATE, TRANSFORM & RECOVER RECOMBINANT PROGENY

pVL1392/sKK-1 #1

P_poth

BglII    PstI    HindIII    XmaIII BamHI

pre-KK-0    pro-KK-1    KK-1